# EUROPÄISCHE PATENTSCHRIFT

(11) **EP 3 610 899 B1**
(45) Veröffentlichungstag und Bekanntmachung des Hinweises auf die Patenterteilung: **01.11.2023**
(21) Anmeldenummer: 19199460.7
(22) Anmeldetag: 18.09.2017
(51) Int. Cl.: A61M 1/00, A61M 3/02, F04B 43/12

(54) **ABSAUG- UND ZUFÜHRVORRICHTUNG MIT ANTRIEBSEINHEIT UND ANSCHLUSSTEIL**
EXTRACTION AND SUPPLY DEVICE WITH DRIVE UNIT AND CONNECTION PIECE
DISPOSITIF D'ASPIRATION ET D'ALIMENTATION COMPRENANT UNE UNITÉ D'ENTRAÎNEMENT ET PARTIE DE RACCORDEMENT

(30) Priorität: 20.09.2016 EP 16189674
(43) Veröffentlichungstag der Anmeldung: 19.02.2020
(62) Teilanmeldung aus: 17768457.8
(73) Patentinhaber: Medela Holding AG, 6340 Baar (CH)
(72) Erfinder: EHLERT, Hilmar, 6052 Hergiswil (CH); BANNWART, Lukas, 6343 Rotkreuz (CH)
(74) Vertreter: Grünecker Patent- und Rechtsanwälte PartG mbB

(56) Entgegenhaltungen:
- WO-A1-94/05346
- WO-A1-2014/082003
- WO-A1-2018/054834
- WO-A2-98/06446
- WO-A2-2006/015301
- US-A1- 2012 302 976
- US-B1- 6 280 440

## Beschreibung

### TECHNISCHES GEBIET

Die vorliegende Erfindung betrifft eine Vorrichtung zum Absaugen von Körperfluiden und zum Zuführen einer Substanz zu einem menschlichen oder tierischen Körper sowie ein Anschlussteil einer derartigen Vorrichtung. Derartige Vorrichtungen werden insbesondere im medizinischen Bereich verwendet, beispielsweise in der mit Instillation oder Irrigation kombinierten Unterdruck-Wundtherapie, in der Augenchirurgie oder bei der Fettabsaugung.

### STAND DER TECHNIK

Im medizinischen Bereich gibt es vielfältige Anwendungen, bei denen einerseits Körperfluide oder Sekrete aus Körperkavitäten oder Wunden mittels einer Pumpe abgesaugt und andererseits eine Substanz dem Körper zugeführt werden. Mögliche Anwendungsgebiete betreffen insbesondere die mit Instillation kombinierte Unterdruck-Wundtherapie, die Augenchirurgie und die Liposuktion (Fettabsaugung). Je nach Anwendung erfolgen die Absaugung und die Zuführung dabei gleichzeitig, nacheinander und/oder abwechslungsweise intermittierend.

Bei der zuzuführenden Substanz kann es sich beispielsweise um eine physiologische oder nicht physiologische Kochsalzlösung, ein Arzneimittel oder um ein Gemisch davon handeln. Die Substanz kann zum Beispiel zur Förderung der Wundheilung, zur Verhinderung von Infektionen oder zur lokalen Anästhesie dienen. Das Zuführen der Substanz kann somit zur Spülung oder therapeutischen, diagnostischen und/oder präventiven Zwecken dienen.

Oft wird zum Zuführen der Substanz ähnlich wie bei der herkömmlichen Infusion ein mit der zuzuführenden Substanz gefüllter Flüssigkeitsbeutel oder eine Flasche erhöht über der zu behandelnden Körperstelle angeordnet, so dass die Substanz aufgrund des hydrostatischen Druckes durch eine Zuführleitung der zu behandelnden Stelle zugeführt wird. Separat dazu werden die Körperfluide von einer Vakuumpumpe via einer entsprechenden Leitung abgesaugt.

Um eine bessere Einstellung und Regelung beim Zuführen der Substanz zu ermöglichen, und/oder um unabhängig von der Anordnung und insbesondere der Höhenlage des mit der Substanz gefüllten Flüssigkeitsbehälters zu sein, sind auch Systeme hinlänglich bekannt, bei denen die Zuführung der Substanz zum Körper mittels einer Pumpe, insbesondere einer sog. Peristaltik- oder Schlauchpumpe erfolgt.

Beispielsweise offenbart die US 2016/0015873 ein Therapiesystem mit einer Instillationskartusche, welche eine Pumpe zum Zuführen von Instillationsflüssigkeit zum Körper aufweist.

Die WO 2016/065335 offenbart ein Gerät mit einem pneumatisch angetriebenen Instillationsregler.

Die WO 2015/091070 offenbart ebenso wie die US 2008/0154184 , die US 2008/0154182, die US 8,591,453 und die US 2008/0154185 ein Gerät mit zwei in einem gemeinsamen Gehäuse angeordneten Pumpen, von denen eine zum Absaugen von Körperfluiden und die andere zum Zuführen einer Substanz dient.

In der US 2014/0163487 ist ein Gerät mit zwei Pumpen offenbart, wobei hier der Pumpenkopf einer Peristaltikpumpe, welche zum Zuführen einer Substanz zum Körper dient, auf der Aussenseite des Pumpaggregatgehäuses angeordnet ist. Ein Flüssigkeitsbehälter, welcher zur Aufnahme einer Instillationsflüssigkeit dient, ist an das Pumpaggregatgehäuse anschliessbar. Am Flüssigkeitsbehälter ist eine Schlauchführung ausgebildet, aufgrund welcher der Pumpenkopf, wenn der Behälter am Pumpaggregatgehäuse angeschlossen ist, eine entsprechende Pumpwirkung auf den aus dem Behälterinneren herausführenden Instillationsschlauch ausübt, um so die Instillationsflüssigkeit zum Körper hin zu pumpen.

Diese Vorrichtungen, welche einerseits zum Absaugen von Körperfluiden sowie andererseits zum Zuführen einer Substanz dienen, sind auf bestimmte Anwendungen eingeschränkt und zudem meist aufwändig und entsprechend kostenintensiv in der Herstellung.

Die EP 0 293 081 offenbart eine Vorrichtung mit einem Antriebsgerät, das einen aussenseitig angeordneten Pumpenkopf aufweist. Eine Kassette mit einer Schlauchführung ist derart an das Antriebsgerät anschliessbar, dass der Pumpenkopf in Kombination mit einer in der Schlauchführung geführten Sekretleitung eine Peristaltikpumpe bildet. Mittels der Peristaltikpumpe sind Sekrete aus dem Wundbereich durch die Sekretleitung hindurch absaugbar. Eine Infusionsleitung für eine herkömmliche Infusion ist durch die Kassette hindurchgeführt.

In den Dokumenten FR 2 624 378 , FR2624376 und FR2624377 sind Instillationsbehälter zum Zuführen einer Instillationsflüssigkeit offenbart. Im Inneren der starr ausgebildeten Behälter ist jeweils ein flexibel ausgebildeter Beutel angeordnet, in welchem Instillationsftüssigkeit aufgenommen ist. Mittels Erzeugen eines Druckes im Behälterinneren kann die Instillationsflüssigkeit aus dem Beutel hinaus befördert werden.

Weitere gattungsgemässe Vorrichtungen sind zum Beispiel in der WO 2011/018132 und der US 2016/0095964 offenbart.

Die US 2012/302976 A1 offenbart eine gattungsgemäße Vorrichtung. Die WO 2006/015301 A2 offenbart eine Vorrichtung zum Absaugen von Körperfluiden und zum Zuführen einer fluiden Substanz zu einem menschlichen oder tierischen Körper, wobei eine Antriebseinheit einen Pumpenkopf umfasst, der nach oben weist und vom Schlauch umschlungen wird. Die WO 94/05346 A1 offenbart ein Kassettensystem zur Steuerung der Spülung einer Absaugflüssigkeit zu und von einem chirurgischen Instrument. Die WO 2014/082003 A1 offenbart eine Therapievorrichtung zur Instillation von Flüssigkeit an einer Gewebestelle.

### DARSTELLUNG DER ERFINDUNG

Es ist deshalb eine Aufgabe der Erfindung, eine vielseitig verwendbare Vorrichtung zum Absaugen von Körperfluiden und zum Zuführen einer Substanz zu einem menschlichen oder tierischen Körper zu schaffen, welche zudem günstig herstellbar ist. Die Vorrichtung sollte ausserdem für den Anwender einfach handhabbar sein.

Zur Lösung dieser Aufgabe wird eine Vorrichtung vorgeschlagen, wie sie im Anspruch 1 angegeben sind. Ausserdem wird im Anspruch 9 ein Anschlussteil einer derartigen Vorrichtung angegeben. Vorteilhafte Ausgestaltungen der Erfindung sind in den abhängigen Ansprüchen angegeben.

Die vorliegende Erfindung stellt also eine Vorrichtung zum Absaugen von Körperfluiden und zum Zuführen einer Substanz zu einem menschlichen oder tierischen Körper zur Verfügung, aufweisend
eine Antriebseinheit mit einem Antrieb,
einen vom Antrieb antreibbaren Pumpenkopf, sowie
ein lösbar, das heisst bevorzugt ohne Zuhilfenahme eines Werkzeugs, an die Antriebseinheit anschliessbares Anschlussteil mit einer Schlauchführung, welche derart zur Aufnahme eines Schlauches ausgebildet ist, dass der Schlauch, wenn das Anschlussteil bestimmungsgemäss an der Antriebseinheit angeschlossen ist, in Kombination mit dem Pumpenkopf eine Peristaltikpumpe bildet, mit welcher die fluide Substanz durch den Schlauch hindurch zum menschlichen oder tierischen Körper beförderbar ist.

Beim Anschlussteil handelt es sich um einen Fluidsammelbehälter zum Sammeln der abgesaugten Körperfluide oder um ein Teil eines solchen Fluidsammelbehälters zum Sammeln der abgesaugten Körperfluide oder um ein Zwischenteil, welches an einen solchen Fluidsammelbehälter zum Sammeln der abgesaugten Körperfluide anschliessbar ist, um eine Verbindung zwischen der Antriebseinheit und dem Fluidsammelbehälter herzustellen. Im Falle eines Zwischenteils dient dieses also insbesondere dazu, den Fluidsammelbehälter funktionell mit der Antriebseinheit zu verbinden.

Die Antriebseinheit weist ein Gehäuse auf, in welchem der Pumpenkopf angeordnet ist. Erfindungsgemäß ist vorgesehen, dass der Pumpenkopf zumindest mit einem Teil seiner Umfangsfläche durch eine Seitenwand des Gehäuses hindurch nach haussen zum Anschlussteil hin vorragt oder das Gehäuse im Bereich des Pumpenkopfes eine Stufe mit einer Fläche aufweist, die vom Pumpenkopf durchragt wird.

Der Pumpenkopf kann im Anschlussteil integriert sein. Falls der Pumpenkopf im Anschlussteil integriert ist, kann, da der Fluidsammelbehälter üblicherweise nach einer gewissen Benutzungsdauer, oft sogar nach einmaligem Gebrauch, entsorgt bzw. ausgewechselt wird, bei einer Ausbildung des Anschlussteils als Fluidsammelbehälter bzw. als Teil davon sichergestellt werden, dass auch der Pumpenkopf bereits dann ersetzt wird. Die Anforderungen zur Herstellung des Pumpenkopfes werden dadurch erheblich verringert, so dass die Vorrichtung insgesamt günstiger herstellbar ist.

Der Pumpenkopf kann im Anschlussteil integriert sein, und die Antriebseinheit kann ein mit dem Antrieb verbundenes Kopplungselement aufweisen, welches den Pumpenkopf an den Antrieb koppelt, wenn das Anschlussteil bestimmungsgemäss an der Antriebseinheit angeschlossen ist. Beim Anschliessen des Anschlussteils an die Antriebseinheit kommt der Pumpenkopf dann derart im Bereich der Schlauchführung zu liegen, dass er in Kombination mit einem in die Schlauchführung eingelegten Schlauch eine Peristaltikpumpe bildet. Der Pumpenkopf kann unabhängig davon im Anschlussteil integriert sein und die Antriebseinheit kann unabhängig davon ein mit dem Antrieb verbundenes Kopplungselement zur Koppelung des Pumpenkopfes an den Antrieb aufweisen, ob das Anschlussteil als Fluidsammelbehälter, als Teil eines Fluidsammelbehälters oder als Zwischenteil ausgebildet ist oder nicht.

Bei einer Integration des Pumpenkopfes im Anschlussteil ist die Vorrichtung wesentlich vielseitiger verwendbar. So können zum Beispiel verschiedenartige Anschlussteile mit unterschiedlichen Pumpenköpfen an dieselbe Antriebseinheit angeschlossen werden. Der Pumpenkopf kann bzgl. seiner Dimensionierung und Ausgestaltung spezifisch an das jeweilige Anschlussteil angepasst sein. Die Anschlussteile können zum Beispiel unterschiedliche Substanzen enthalten und/oder für unterschiedliche Anwendungen dienen. Mittels entsprechender Auslegung des Pumpenkopfes können unterschiedliche Erfordernisse problemlos und unter Verwendung von immer derselben Antriebseinheit erfüllt werden.

Des Weiteren sind die Anforderungen an das Anschlussteil bzgl. dessen Lebensdauer oft weniger hoch als die entsprechenden Anforderungen an die Antriebseinheit. Bei einer Integration des Pumpenkopfes im Anschlussteil gelten die geringeren Anforderungen bzgl. Lebensdauer auch für den Pumpenkopf, welcher somit erheblich günstiger herstellbar ist, als bei einer Vorrichtung mit einem in der Antriebseinheit integriertem Pumpenkopf.

Die erfindungsgemässe Vorrichtung wird für medizinische Zwecke eingesetzt, insbesondere zu der mit Instillation oder Irrigation kombinierten Unterdruckbehandlung von Wunden am menschlichen oder tierischen Körper. Andere Anwendungsgebiete sind jedoch möglich, beispielsweise die kombinierte Fettabsaugung und Spülung bei der Liposuktion oder das Spülen von Kathetern zur Vermeidung von Verstopfungen oder die kombinierte Absaugung und Spülung bei der Augenchirurgie.

Die Vorrichtung ist bevorzugt zum gleichzeitigen Absaugen von Körperfluiden und Zuführen einer Substanz geeignet.

Peristaltikpumpen sind auch unter dem Begriff Schlauchpumpen bekannt und eignen sich besonders gut um auch geringe Fluidvolumina einer Substanz, insbesondere einer fluiden Substanz wie einer Flüssigkeit, kontrolliert zum Körper zu befördern. Bei Peristaltikpumpen kann zudem eine Kontamination der fluiden Substanz und der Vorrichtung ausgeschlossen werden. Eine Peristaltikpumpe weist in der Regel zumindest einen drehbar gelagerten Pumpenkopf sowie ein in einem Schlauchbett gelagerten Schlauch auf. In der Regel bildet die Schlauchführung das Schlauchbett der Peristaltikpumpe. Am Pumpenkopf sind üblicherweise zum Beispiel Anpressrollen oder Gleitschuhe angebracht, welche bei einer Drehung des Pumpenkopfes den im Schlauchbett gelagerten Schlauch mechanisch verformen und dadurch eine Substanz durch den Schlauch hindurch befördern.

Beispielsweise kann eine Instillationsleitung in die am Anschlussteil ausgebildete Schlauchführung derart eingelegt werden, dass sie zumindest teilweise um den Pumpenkopf herum geführt ist, so dass dieser den Schlauch der Instillationsleitung mechanisch verformen und eine darin enthaltene Substanz befördern kann. Die Substanz kann zum Beispiel in einem oberhalb der Peristaltikpumpe angeordneten Flüssigkeitsbeutel oder in einer entsprechend angeordneten Flasche gelagert sein und daraus zugeführt werden.

Beim Antrieb handelt es sich in der Regel um einen Motor, insbesondere um einen Elektromotor. In einer insbesondere bevorzugten Ausführungsform handelt es sich um einen bürstenlosen Gleichstrommotor, da ein solcher in der Regel bei tiefen Drehzahlen von weniger als 100U/min betrieben werden kann. Ein bürstenloser Gleichstrommotor lässt zudem eine Druckregelung mit relativ kleiner Amplitude zu, wodurch eine sehr genaue Steuerung des Druckes (Unterdruck oder Überdruck) möglich wird.

Beim Anschlussteil kann es sich auch um einen Behälter handeln, der sowohl zum Sammeln der abgesaugten Körperfluide als auch zur Bereitstellung der fluiden Substanz dient. Üblicherweise ist dann eine Instillationsleitung, welche in das Innere des Behälters mündet und somit aus diesem herausführt, in die am Anschlussteil vorgesehene Schlauchführung eingelegt, so dass mittels der Peristaltikpumpe die fluide Substanz durch die Instillationsleitung aus dem Instillationsbehälter heraus beförderbar ist.

Ein Behälter, der sowohl zum Sammeln der abgesaugten Körperfluide als auch zur Bereitstellung der fluiden Substanz dient, weist bevorzugt einen Innenraum auf, der in einen ersten Bereich zum Sammeln der abgesaugten Körperfluide sowie in einen zweiten Bereich zur Bereitstellung der fluiden Substanz unterteilt ist. Die beiden Bereiche können mittels einer starren Trennwand voneinander getrennt sein. Bevorzugt sind sie jedoch mittels einer flexibel ausgebildeten Trennwand voneinander getrennt. Der Behälter kann insbesondere ein kombinierter Fluidsammel- und Instillationsbehälter sein. Die flexibel ausgebildete Trennwand bewirkt, dass eine Verkleinerung des zweiten Bereiches unmittelbar eine Vergrösserung des ersten Bereiches bewirkt. Das Gesamtvolumen des Innenraums, welches vorteilhaft im Wesentlichen der Summe der Volumen des ersten und des zweiten Bereiches entspricht, bleibt dabei konstant. Im laufenden Betrieb der Vorrichtung füllt sich der erste Bereich üblicherweise allmählich mit den abgesaugten Körperfluiden. Während des Zuführens der fluiden Substanz zum Körper leert sich der zweite Bereich allmählich, wodurch sich sein Volumen verringert. Die flexiblen Volumina des ersten und des zweiten Bereiches ermöglichen eine erhebliche Platzeinsparung im Vergleich zum Beispiel zu einer Lösung mit einem ersten starr ausgebildeten Behälter zum Sammeln der abgesaugten Körperfluide sowie einem zweiten starr ausgebildeten Behälter zur Bereitstellung der fluiden Substanz. Im Idealfall, wenn die fluide Substanz schneller oder gleich schnell aus dem Behälter abgeführt wird wie Körperfluide angesaugt werden, kann das Volumen des Behälters dadurch sogar halbiert werden.

Der erwähnte Behälter mit einem Innenraum, der mittels einer flexiblen Trennwand in einen ersten Bereich zum Sammeln der abgesaugten Körperfluide sowie in einen zweiten Bereich zur Bereitstellung der fluiden Substanz unterteilt ist, stellt eine weitere unabhängige Erfindung dar. Ein derartiger Behälter kann auch bei herkömmlichen Vorrichtungen, insbesondere herkömmlichen Instillations- oder Irrigationsvorrichtungen, eingesetzt werden, bei welchen einerseits Körperfluide abgesaugt und andererseits eine fluide Substanz einem menschlichen oder tierischen Körper zugeführt wird. Der Behälter muss nicht zwingend eine Schlauchführung, welche mit einem Pumpenkopf zusammenwirkt, aufweisen. Der Behälter kann zum Beispiel auch nur via Schlauchverbindungen an ein Pumpaggregat angeschlossen werden, und die fluide Substanz kann auch ohne die Hilfe einer Pumpe dem Körper zugeführt werden, indem zum Beispiel mittels Anordnung des Behälters oberhalb des Körpers die Schwerkraft ausgenutzt wird.

Vorteilhaft wird die flexible Trennwand durch einen im Innenraum angeordneten Beutel gebildet. Der Beutel trennt dann also den ersten Bereich vom zweiten Bereich. Bevorzugt begrenzt der Beutel den ersten oder den zweiten Bereich, vorzugsweise jedoch den zweiten Bereich, zu einem überwiegenden Grossteil. Der Beutel dient dann also vorzugsweise zur Bereitstellung der fluiden Substanz.

Der Behälter, welcher sowohl zum Sammeln der abgesaugten Körperfluide als auch zur Bereitstellung der fluiden Substanz dient und zwei mittels einer flexiblen Trennwand getrennte Bereiche hat, weist vorteilhaft einen Vakuumanschluss zum Anschliessen einer Vakuumleitung auf, um im ersten Bereich des Innenraums ein Vakuum zu erzeugen. Ausserdem weist der Behälter vorteilhaft einen Sekretleitungsanschluss zum Anschliessen einer zum menschlichen oder tierischen Körper führenden Sekretleitung auf, um mittels des im ersten Bereich des Innenraums erzeugten Vakuums die Körperfluide durch die Sekretleitung hindurch in den ersten Bereich hinein zu saugen. Des Weiteren weist der Behälter vorzugsweise einen Anschluss zum Anschliessen einer Zuführleitung auf, um die fluide Substanz vom zweiten Bereich des Innenraums aus durch die Zuführleitung hindurch dem menschlichen oder tierischen Körper zuzuführen.

Insbesondere wenn es sich beim Anschlussteil um einen kombinierten Fluidsammel- und Instillationsbehälter handelt, aber auch in anderen Fällen, können das Anschlussteil ein Identifikationsmerkmal und die Antriebseinheit eine Identifikationseinheit aufweisen, um zu identifizieren, welche Art eines Anschlussteils an der Antriebseinheit angeschlossen ist. Die Antriebseinheit kann dann insbesondere dazu ausgebildet sein, in Abhängigkeit des identifizierten Anschlussteils einen von mehreren möglichen Betriebsmodi zum Antreiben der Peristaltikpumpe auszuwählen oder vorauszuwählen. Im Falle, dass es sich beim Anschlussteil um einen kombinierten Fluidsammel- und Instillationsbehälter handelt, kann das Identifikationsmerkmal insbesondere zur Identifikation der Art der im Behälter enthaltenen Substanz dienen.

In einer insbesondere bevorzugten Ausführungsform handelt es sich beim Anschlussteil um ein Wegwerfteil, das für einen einmaligen Gebrauch ausgelegt ist und vorteilhaft im Wesentlichen vollständig aus Spritzgussteilen hergestellt ist. Bei einem Wegwerfteil, welches nach einer gewissen Zeit ausgetauscht und entsorgt wird, zum Beispiel wenn der Fluidsammelbehälter voll oder der Instillationsbehälter leer ist, sind die Anforderungen insbesondere an einen allfällig darin integrierten Pumpenkopf verhältnismässig gering. Die Herstellungskosten für die gesamte Vorrichtung können dadurch erheblich gesenkt werden im Vergleich zu einer Vorrichtung, bei welcher der Pumpenkopf im oder am Pumpaggregatgehäuse angeordnet ist und dadurch für eine viel längere Lebensdauer ausgelegt sein muss.

Um die Drehgeschwindigkeit des Pumpenkopfes gegenüber derjenigen des Antriebs anzupassen, kann ein Getriebe, insbesondere ein Planetengetriebe vorgesehen sein, via welches der Pumpenkopf an den Antrieb gekoppelt ist, wenn das Anschlussteil bestimmungsgemäss an der Antriebseinheit angeschlossen ist. Das Getriebe kann insbesondere im Anschlussteil integriert sein.

Die Antriebseinheit kann ausserdem eine Vakuumpumpe, insbesondere eine Membranpumpe, aufweisen, welche zum Absaugen der Körperfluide dient. Vorteilhaft bildet die Vakuumpumpe einen Teil der Antriebseinheit. Eine Membranpumpe weist in der Regel zumindest eine Membran sowie eine von dieser begrenzte Pumpkammer auf.

Die Antriebseinheit weist bevorzugt ein Gehäuse auf, in welchem sowohl der Antrieb als auch die Vakuumpumpe angeordnet sind. Das Kopplungselement ist vorteilhaft auf einer Aussenseite des Gehäuses angeordnet.

In einer bevorzugten Ausführungsform dient derselbe Antrieb, welcher zum Antreiben der Peristaltikpumpe dient, auch zum Antreiben der Vakuumpumpe. Indem derselbe Antrieb zum Antreiben von beiden Pumpen dient, kann die Vorrichtung insgesamt kleiner dimensioniert werden und zudem ein geringeres Gewicht aufweisen. Die Vorrichtung kann dadurch insbesondere derart ausgebildet werden, dass sie tragbar ist, das heisst dass sie von einem Benutzer alleine und ohne übermässigen Kraftaufwand bequem getragen werden kann. Vorteilhaft weist die Vorrichtung zudem eine insgesamt kompakte Bauweise auf. Aufgrund des nur einen Antriebs ist auch die Fehleranfälligkeit verringert, und die Vorrichtung ist insgesamt kostengünstiger herstellbar.

Bevorzugt ist der Pumpenkopf über zumindest einen Freilauf an den Antrieb gekoppelt, wenn das Anschlussteil bestimmungsgemäss an der Antriebseinheit angeschlossen ist. Der Freilauf ist in der Regel im Pumpaggregatgehäuse untergebracht, könnte grundsätzlich aber auch im Anschlussteil integriert sein. Falls derselbe Antrieb sowohl zum Antreiben der Peristaltikpumpe als auch zum Antreiben einer Vakuumpumpe dient, kann der Freilauf insbesondere bewirken, dass je nach Drehrichtung des Antriebs entweder beide Pumpen zusammen angetrieben werden, oder dass nur die Peristaltikpumpe bzw. nur die Vakuumpumpe angetrieben wird. Dies kann für bestimmte Anwendungen erwünscht sein.

Ein erster Freilauf kann zum Beispiel die Peristaltikpumpe an den Antrieb koppeln. In bestimmten Ausführungsformen kann zudem ein zweiter Freilauf vorhanden sein, mittels welchem die Vakuumpumpe an den Antrieb gekoppelt ist. Der zweite Freilauf weist vorteilhaft eine im Vergleich zum ersten Freilauf entgegengesetzte Freilaufrichtung auf. Es kann dadurch insbesondere sichergestellt werden, dass je nach Drehrichtung des Antriebs entweder die erste Pumpe oder die zweite Pumpe angetrieben wird, nicht jedoch beide zusammen. Das heisst, entweder wird eine Substanz zum Körper zugeführt, oder es werden Körperflüssigkeiten abgesaugt, jedoch nicht beides gleichzeitig.

Beim ersten und, falls vorhanden, zweiten Freilauf kann es sich beispielsweise um einen Klemmkörper-Freilauf, um eine Schlingfederkupplung (Federwickel-Freilauf) oder um eine selbstsynchronisierende Schaltkupplung handeln.

Insbesondere wenn der Antrieb sowohl zum Antreiben der Peristaltikpumpe als auch zum Antreiben der Vakuumpumpe dient, aber auch wenn der Antrieb nur zum Antreiben der Peristaltikpumpe dient, kann die Vorrichtung zusätzlich ein Ventil, insbesondere ein Pneumatikventil, aufweisen, mittels welchem die Vakuumpumpe mit der Umgebung verbindbar ist, um zumindest teilweise oder sogar vollständig anstatt Körperfluide Luft aus der Umgebung anzusaugen. Dies führt zu einer Reduzierung oder sogar Eliminierung der Sauwirkung der Vakuumpumpe im System. Das Ventil kann insbesondere mit einem Vakuumanschluss der Vakuumpumpe verbunden sein, an welchen eine Saugleitung anschliessbar ist. Mittels Umstellen des Ventils kann der Vakuumanschluss bei Bedarf zumindest teilweise oder sogar vollständig statt mit der Saugleitung mit der Umgebung verbunden werden. Das Ventil ermöglicht es, die Saugleistung zum Absaugen der Körperfluide bei gleichbleibender Motorleistung zu variieren.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine perspektivische Ansicht eines schematisch gezeigten Pumpaggregatgehäuses einer Vorrichtung gemäss einer Ausführungsform, wobei die Vorderwand weggelassen ist;
- Fig. 2: eine erste perspektivische Ansicht eines Fluidsammelbehälters der zum Anschliessen an das Pumpaggregatgehäuse der Fig. 1 geeignet ist;
- Fig. 3: eine perspektivische Ansicht des Fluidsammelbehälters der Fig. 2, wobei die Abdeckung des Pumpenkopfes weggelassen ist;
- Fig. 4: eine erste perspektivische Ansicht von nur dem Pumpenkopf sowie der Instillationsleitung des Fluidsammelbehälters der Fig. 2;
- Fig. 5: eine perspektivische Ansicht von Teilen des Pumpenkopfes sowie der Instillationsleitung des Fluidsammelbehälters der Fig. 2;
- Fig. 6: eine zweite perspektivische Ansicht des Fluidsammelbehälters der Fig. 2;
- Fig. 7: eine zweite perspektivische Ansicht von nur dem Pumpenkopf sowie der Instillationsleitung des Fluidsammelbehälters der Fig. 2;
- Fig. 8: eine erste perspektivische Teilansicht auf den mit einer Abdeckung abgedeckten Bereich des Pumpenkopfes eines Fluidsammelbehälters gemäss einer Variante;
- Fig. 9: eine perspektivische Teilansicht auf den Bereich des zumindest teilweise als Planetengetriebe ausgebildeten Pumpenkopfes des Fluidsammelbehälters der Fig. 8, ohne Abdeckung;
- Fig. 10: eine perspektivische Teilansicht auf den Bereich des Pumpenkopfes des Fluidsammelbehälters der Fig. 8, ohne Planetengetriebe und ohne Abdeckung;
- Fig. 11: eine erste perspektivische Ansicht auf das zumindest teilweise den Pumpenkopf bildende Planetengetriebe des Fluidsammelbehälters der Fig. 8, mit Instillationsleitung;
- Fig. 12: eine zweite perspektivische Teilansicht auf den Bereich des Pumpenkopfes des Fluidsammelbehälters der Fig. 8;
- Fig. 13: eine zweite perspektivische Ansicht auf das zumindest teilweise den Pumpenkopf bildende Planetengetriebe des Fluidsammelbehälters der Fig. 8, mit Instillationsleitung;
- Fig. 14: eine perspektivische Ansicht einer schematisch gezeigten Vorrichtung gemäss einer weiteren erfindungsgemässen Ausführungsform;
- Fig. 15: eine erste perspektivische Ansicht auf die Peristaltikkassette der Vorrichtung der Fig. 14, mit daran angebrachtem Aufhängbügel;
- Fig. 16: eine perspektivische Ansicht des Fluidsammelbehälters der Vorrichtung der Fig. 14;
- Fig. 17: eine perspektivische Ansicht des Pumpaggregatgehäuses der Vorrichtung der Fig. 14;
- Fig. 18: eine zweite perspektivische Ansicht der Peristaltikkassette der Vorrichtung der Fig. 14, mit daran angebrachtem Aufhängbügel sowie am Aufhängbügel angehängtem Flüssigkeitsbehälter;
- Fig. 19: eine perspektivische Ansicht einer schematisch gezeigten Vorrichtung gemäss einer nicht erfindungsgemässen Ausführungsform;
- Fig. 20: eine erste perspektivische Ansicht des Instillationsbehälters der Vorrichtung der Fig. 19;
- Fig. 21: eine perspektivische Ansicht des Instillationsbehälters der Vorrichtung der Fig. 19, wobei die Abdeckung des Pumpenkopfes weggelassen ist,
- Fig. 22: eine perspektivische Ansicht des Basisteils des Instillationsbehälters der Vorrichtung der Fig. 19;
- Fig. 23: eine zweite perspektivische Ansicht des Instillationsbehälters der Vorrichtung der Fig. 19;
- Fig. 24: eine perspektivische Ansicht einer schematisch gezeigten Vorrichtung gemäss einer weiteren erfindungsgemässen Ausführungsform;
- Fig. 25: eine perspektivische Ansicht des Pumpaggregatgehäuses der Vorrichtung der Fig. 24, wobei das Gehäuse selbst nur mit gestrichelten Linien angedeutet ist;
- Fig. 26: eine perspektivische Ansicht des Fluidsammelbehälters der Vorrichtung der Fig. 24;
- Fig. 27: eine perspektivische Ansicht des Pumpaggregatgehäuses der Vorrichtung der Fig. 24;
- Fig. 28: eine erste perspektivische Ansicht eines Fluidsammelbehälters einer Vorrichtung gemäss einer weiteren erfindungsgemässen Ausführungsform;
- Fig. 29: eine perspektivische Ansicht eines zur selben Vorrichtung wie der Fluidsammelbehälter der Fig. 28 gehörenden Pumpaggregatgehäuses;
- Fig. 30: eine perspektivische Ansicht des Fluidsammelbehälters der Fig. 28, mit abgenommenem Basisteil;
- Fig. 31: eine perspektivische Ansicht des Basisteils des Fluidsammelbehälters der Fig. 28;
- Fig. 32: eine zweite perspektivische Ansicht des Fluidsammelbehälters der Fig. 28, wobei das transparente Teil nur mit gestrichelten Linien angedeutet ist, im Zustand zu Beginn einer Behandlung; sowie
- Fig. 33: eine perspektivische Ansicht des Fluidsammelbehälters der Fig. 28, wobei das transparente Teil nur mit gestrichelten Linien angedeutet ist, im Zustand nach einer gewissen Behandlungsdauer.

### BESCHREIBUNG BEVORZUGTER AUSFÜHRUNGSFORMEN

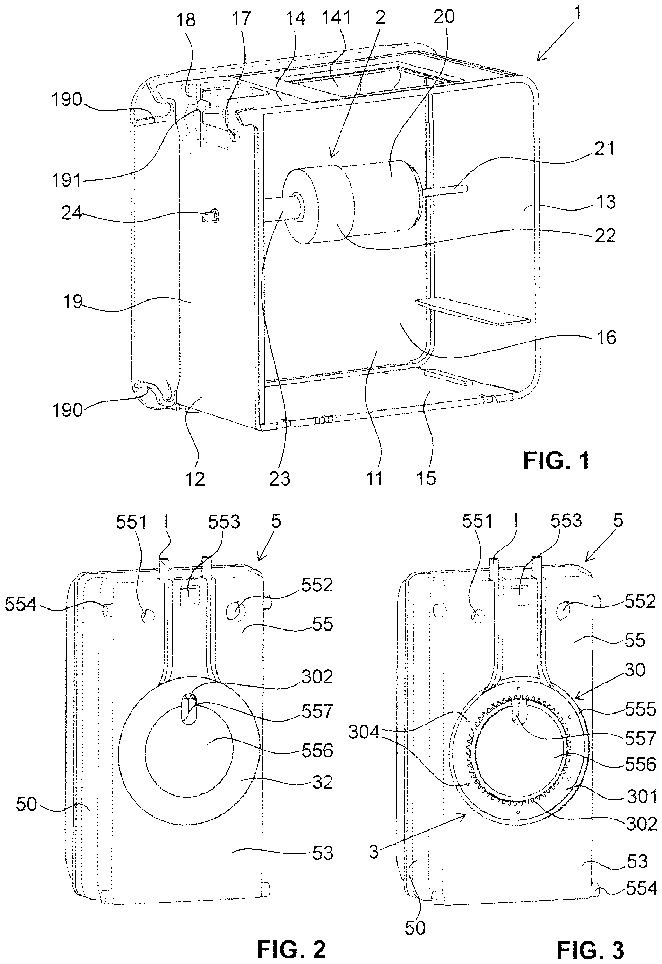

In den Figuren 1 bis 33 sind unterschiedliche Ausführungsformen von Vorrichtungen gezeigt. Die in den Figuren 1 bis 33 gezeigten Vorrichtungen sind insbesondere zur kombinierten Unterdruck- und Instillations-/Irrigationsbehandlung von Wunden am menschlichen oder tierischen Körper geeignet. Dementsprechend beziehen sich die nachfolgenden Erläuterungen jeweils auf die Verwendung der Vorrichtungen in der kombinierten Unterdruck- und Instillations-/Irrigationsbehandlung. Es wäre grundsätzlich aber auch möglich, diese Vorrichtungen, bei entsprechend angepasster Auslegung, für die Katheterspülung, die Augenchirurgie, die Fettabsaugung oder eine andere medizinische Anwendung zu verwenden.

Elemente mit einer identischen oder ähnlichen technischen Funktion und Wirkung sind in den Figuren 1 bis 33 bei den verschiedenen Ausführungsformen jeweils mit denselben Bezugszeichen versehen oder weisen dasselbe, jedoch mit einem Strich (') versehene Bezugszeichen auf.

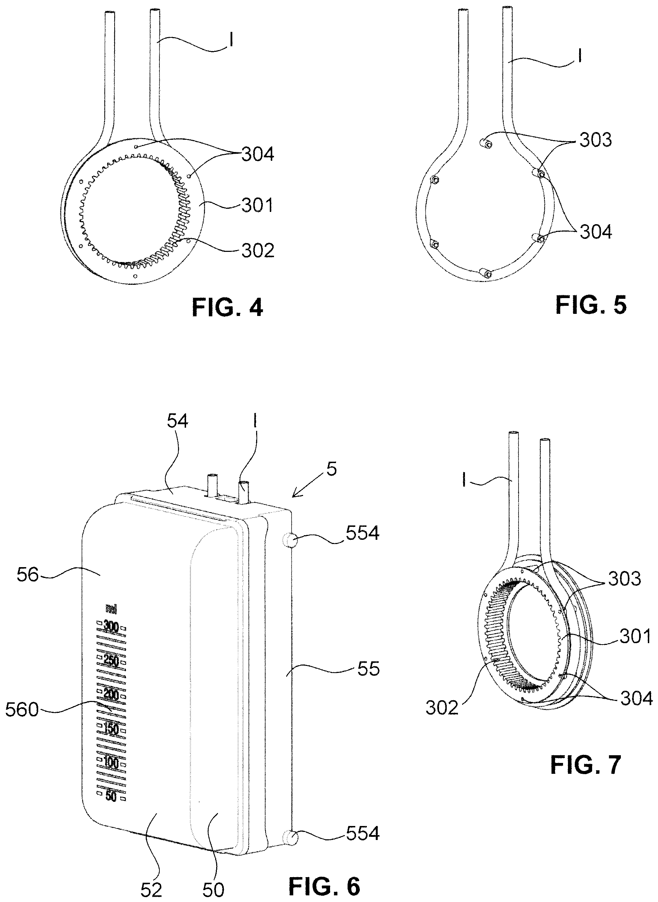

Die Vorrichtung der ersten Ausführungsform, welche in den Figuren 1 bis 7 gezeigt ist, weist ein Pumpaggregatgehäuse 1 (Figur 1) mit einem daran anschliessbaren Anschlussteil in Form eines Fluidsammelbehälters 5 (Figuren 2 und 6) auf.

Das Pumpaggregatgehäuse 1 hat eine insgesamt im Wesentlichen quaderförmige Gestalt mit einer in der Figur 1 nicht gezeigten Vorderwand, einer Rückwand 11, einer ersten Seitenwand 12 und einer zweiten Seitenwand 13 sowie einer oberen Wand 14 und einer unteren Wand 15. Die Vorderwand und die Rückwand 11 weisen je eine Wandkante auf, welche der dazwischen angeordneten ersten Seitenwand 12 vorstehen. Der Fluidsammelbehälter 5 ist zwischen diesen Wandkanten gehalten und dadurch einfach, aber trotzdem sicher und geschützt an das Pumpaggregatgehäuse 1 befestigbar.

Zum Einhängen und Halten des Fluidsammelbehälters 5 am Pumpaggregatgehäuse 1 sind am Pumpaggregatgehäuse 1 Aufnahmehaken 190 vorgesehen, in welche entsprechend ausgebildete und angeordnete Zapfen 554 des Fluidsammelbehälters 5 eingreifen können. Mittels einer an einem federbelasteten Element angebrachten Rückhaltenase 191, welche zum Einschnappen in eine am Fluidsammelbehälter 5 ausgebildete Rastkerbe 553 ausgebildet ist, wird der Fluidsammelbehälter 5 am Pumpaggregatgehäuse 1 gesichert. Um die Rastverbindung zwischen der Rückhaltenase 191 und der Rastkerbe 553 zu lösen, kann das federbelastete Element, an welchem die Rückhaltenase 191 angebracht ist, entgegen der Federkraft nach unten gedrückt werden.

Die vorstehenden Wandkanten der Vorderwand und der Rückwand 11 sowie die Aufnahmehaken 190 und die Rückhaltenase 191 bilden gemeinsam eine Behälteraufnahme 19 des Fluidsammelbehälters 5.

Das Pumpaggregatgehäuse 1 weist einen gehäuseseitigen Vakuumanschluss 17 auf, welcher beim Anbringen des Fluidsammelbehälters 5 an das Pumpaggregatgehäuse 1 an einen entsprechend am Fluidsammelbehälter 5 vorgesehenen behälterseitigen Vakuumanschluss 551 gekoppelt wird, so dass via die Anschlüsse 17 und 551 im Inneren des Fluidsammelbehälters 5 ein Vakuum erzeugbar ist, um via eine in den Figuren nicht gezeigte Sekretleitung Köperfluide anzusaugen und im Fluidsammelbehälter 5 zu sammeln. Die Sekretleitung verbindet den Fluidsammelbehälter 5 mit einer Kavität oder Wunde eines Patienten, aus welcher Körperfluide abgesaugt werden sollen,.

Innerhalb der ersten Seitenwand 12 ist ausserdem eine Adapteraufnahme 18 vorgesehen, welche zur Aufnahme eines in den Figuren nicht gezeigten Schlauchadapters dient. Der Schlauchadapter verbindet die Sekretleitung via einen am Fluidsammelbehälter 5 vorgesehenen behälterseitigen Sekretanschluss 552 mit dem Inneren des Fluidsammelbehälters 5.

In einem Innenraum 16 des Pumpaggregatgehäuses 1 ist ein Antriebsstrang 2 mit einem Motor 20 und einer mit dem Motor 20 verbundenen Motorwelle 21 untergebracht. Das Pumpaggregatgehäuse 1 bildet zusammen mit dem Antriebsstrang 2 sowie mit allenfalls weiteren im Innenraum 16 angeordneten Elementen eine Antriebseinheit.

Die Motorwelle 21 treibt mit einem ersten Endbereich direkt eine in der Figur 1 aus darstellerischen Gründen nicht gezeigte Membranpumpe an. Die Membranpumpe ist im Innenraum 16 des Pumpaggregatgehäuses 1 angeordnet. Mit einem zweiten Endbereich, welcher in der Figur 1 nicht sichtbar ist, ist die Motorwelle 21 mit einem Freilauf und/oder Getriebe 22 verbunden, welches die Motorwelle 21 mit einer Antriebswelle 23 verbindet. Die Antriebswelle 23, welche sich entlang der Rotationsachse der Motorwelle 21 erstreckt, ragt durch die erste Seitenwand 12 hindurch.

Auf der Aussenseite des Pumpaggregatgehäuses 1 ist ein Kopplungselement 24 drehfest am Ende der Antriebswelle 23 angebracht. Das Kopplungselement 24 hat die Form eines Zahnrades. Im vorliegenden Fall ist es ein Zahnrad mit vier Zähnen. Via das Kopplungselement 24 kann ein im Fluidsammelbehälter 5 integrierter Pumpenkopf 30 einer Peristaltikpumpe 3 angetrieben werden, wenn der Fluidsammelbehälter 5 bestimmungsgemäss am Pumpaggregatgehäuse 1 angebracht ist.

Der Motor 20 dient somit sowohl zum Antreiben der Membranpumpe als auch zum Antreiben der Peristaltikpumpe 3. In einer alternativen Ausführungsform können selbstverständlich auch zwei separate Motoren im Innenraum 16 des Pumpaggregatgehäuses vorgesehen sein, wobei einer für den Antrieb der Membranpumpe und der andere für den Antrieb der Peristaltikpumpe 3 dient.

Der Fluidsammelbehälter 5 weist, wie es insbesondere in den Figuren 2 und 6 gut erkennbar ist, eine Vorderwand 50, eine in den Figuren nicht sichtbare Rückwand, zwei Seitenwände 52 und 53 sowie eine obere Wand 54 und eine ebenfalls nicht sichtbare untere Wand auf. Diese Wände werden durch ein aus einem opaken Material hergestellten Basisteil 55 und einem transparenten Teil 56 gebildet. An derjenigen Seitenwand 52, welche ausschliesslich durch das transparente Teil 56 gebildet wird, ist eine Füllstandskala 560 vorgesehen.

Diejenige Seitenwand 53, welche ausschliesslich durch das Basisteil 55 gebildet wird, weist eine zentral angeordnete, ringförmige Vertiefung 555 auf. Innerhalb der ringförmigen Vertiefung 555 bildet die Seitenwand 53 einen konzentrisch angeordneten Lagerzapfen 556, der eine obere, nach oben hin offene Aussparung 557 aufweist.

In der ringförmigen Vertiefung 555 ist ein Hohlrad 301 aufgenommen, welches frei drehbar um den Lagerzapfen 556 herum angeordnet ist. Am Hohlrad 301 sind Anpressrollen 303 in regelmässigen Abständen entlang der Umfangsrichtung frei drehbar um jeweils eine Rollenachse 304 angebracht (siehe Figuren 5 und 7). Die Anpressrollen 303 dienen beim Drehen des Hohlrades 301 zum Abrollen auf einen in der ringförmigen Vertiefung 555 auf der radialen Aussenseite des Hohlrades 301 eingelegten Schlauch. Der Schlauch bildet eine Instillationsleitung I. Indem die Anpressrollen 303 auf dem Schlauch abrollen, wird dieser gegen die radial nach innen gewandte Innenwand der ringförmigen Vertiefung 555 gepresst, so dass eine in der Instillationsleitung I enthaltene fluide Substanz aufgrund der mechanischen Verformung des Schlauches durch diesen hindurchgedrückt und zum Wundbereich hin befördert wird.

Bei der durch die Instillationsleitung I hindurch zu befördernden Substanz kann es sich beispielsweise um eine physiologische oder nicht physiologische Kochsalzlösung, ein Arzneimittel oder um ein Gemisch davon handeln. Die Instillationssubstanz kann zum Spülen einer Wunde oder Kavität dienen. Sie kann aber auch zum Einbringen eines Medikaments oder zur lokalen Betäubung des Wundbereichs dienen.

Auf seiner radialen Innenseite ist am Hohlrad 301 eine umlaufende Verzahnung 302 ausgebildet. Die Verzahnung 302 gerät beim bestimmungsgemässen Anbringen des Fluidsammelbehälters 5 an das Pumpaggregatgehäuse 1 in Eingriff mit dem Kopplungselement 24, so dass bei Drehen der Antriebswelle 23 die Drehbewegung via das Kopplungselement 24 auf das Hohlrad 301 übertragen wird.

Das Hohlrad 301, welches in der Regel mittels einer ringförmigen Abdeckung 32 abgedeckt ist, bildet zusammen mit den daran angebrachten Anpressrollen 303 ein Pumpenkopf 30 einer Peristaltikpumpe 3. Der Pumpenkopf 30 wirkt im Betrieb auf den in die ringförmige Vertiefung 555 eingelegten Schlauch ein, um ein sich darin befindliches Fluid zu befördern. Die ringförmige Vertiefung 555 bildet somit das Schlauchbett der Peristaltikpumpe 3, in welche der die Instillationsleitung I bildende Schlauch eingelegt ist.

Um die Instillationsleitung I zur ringförmigen Vertiefung 555 hin und von dieser wieder weg zu führen, sind innerhalb der ersten Seitenwand 12 entsprechende Führungskanäle zur Aufnahme der Instillationsleitung 1 ausgebildet, welche geradlinig und parallel zueinander von der ringförmigen Vertiefung 555 nach oben bis zur oberen Wand 54 verlaufen. Zusammen mit den Führungskanälen bildet die ringförmige Vertiefung eine Schlauchführung für die Instillationsleitung I.

Vorteilhaft werden im Wesentlichen sämtliche Teile des Fluidsammelbehälters 5, inklusive dem Hohlrad 301 und den daran angebrachten Anpressrollen 303, im Spritzgussverfahren hergestellt. Da der Fluidsammelbehälter 5 üblicherweise ein Wegwerfteil darstellt, welches aus hygienischen Gründen ausgetauscht und entsorgt wird, sobald der Behälter voll ist, sind die Anforderungen an den darin integrierten Pumpenkopf 30 verhältnismässig gering. Die Herstellungskosten für die gesamte Vorrichtung können dadurch erheblich gesenkt werden im Vergleich zu einer Vorrichtung, bei welcher der Pumpenkopf im oder am Pumpaggregatgehäuse angeordnet ist und dadurch für eine viel längere Lebensdauer ausgelegt sein muss.

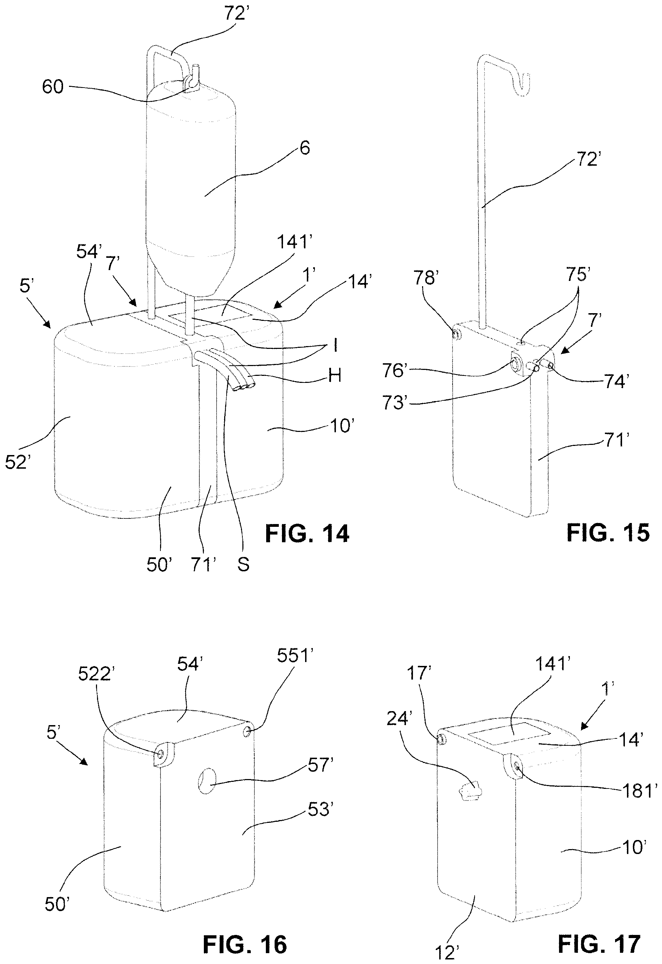

Die Instillationsleitung I kann mit einem Flüssigkeitsbehälter 6, wie demjenigen der in der Figur 14 gezeigt ist, verbunden sein, in welchem eine Instillationsflüssigkeit gelagert ist. Der Flüssigkeitsbehälter 6, welcher insbesondere als Beutel ausgebildet sein kann, kann einen Aufhänger 60 aufweisen, um ihn zum Beispiel an einem Infusionsständer bzgl. der Schwerkraftrichtung oberhalb oder neben der Peristaltikpumpe 3 aufzuhängen.

Im Bereich der oberen Wand 14 des Pumpaggregatgehäuses 1 ist ein Fenster 141 für ein Anzeige- und Bedienfeld angeordnet. Mit Hilfe des Anzeige- und Bedienfeldes kann die Vorrichtung bedient, und es kann insbesondere die Funktion des Motors 20 eingestellt werden. Ausserdem kann das Anzeige- und Bedienfeld zum Anzeigen von Informationen zum Status der Vorrichtung, wie insbesondere aktuelle Pumpleistungen und -zyklen etc., dienen. Das Anzeige- und Bedienfeld kann zum Beispiel als ein Touch Screen ausgebildet sein.

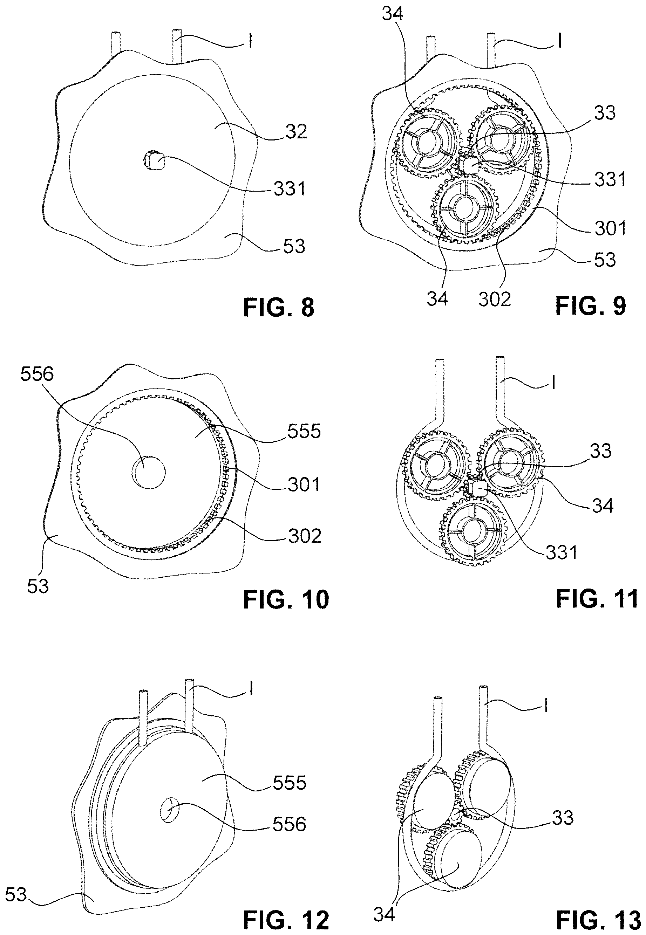

In den Figuren 8 bis 13 ist eine Ausführungsform gezeigt, welche sich nur im Bereich der Peristaltikpumpe 3 von derjenigen der Ausführungsform der Figuren 1 bis 7 unterscheidet:
Die Kopplung zwischen dem Kopplungselement 24 und dem Pumpenkopf 30 erfolgt hier nicht exzentrisch zum Pumpenkopf 30 wie bei der Ausführungsform der Figuren 1 bis 7, sondern zentral. Die Kopplung erfolgt via ein Kopplungselement 331, welches frei drehbar zentral in einer Vertiefung 555 am Fluidsammelbehälter 5 angebracht ist. Das Kopplungselement 331 ist als ein Aussenvierkant mit einem im Wesentlichen quadratischen Querschnitt ausgebildet. Das auf der Seite des Pumpaggregatgehäuses vorgesehene Kopplungselement 24 ist entsprechend komplementär dazu als Innenvierkant ausgebildet, so dass eine Drehbewegung vom Kopplungselement 24 auf das Kopplungselement 331 übertragbar ist.

Das Kopplungselement 331 ist drehfest und zentral an einem Innenrad 33 angebracht. Das Innenrad 33 bildet ein zentral angeordnetes Zahnrad eines Planetengetriebes 33, 34 und steht kämmend mit drei dezentral angeordneten Aussenrädern 34 im Eingriff, welche ebenfalls Teil des Planetengetriebes 33, 34 sind. Die Aussenräder 34 wiederum stehen kämmend mit der Verzahnung 302 des Hohlrades 301 in Eingriff.

Mittels einer Abdeckung 32 ist das gesamte Planetengetriebe 33, 34 sowie das Hohlrad 301 nach aussen hin abdeckbar. Lediglich das Kopplungselement 331 ragt durch eine zentral in der Abdeckung 32 angeordnete Öffnung hindurch nach aussen hin.

Die Aussenräder 34 bilden bei dieser Ausführungsform die Anpressrollen zur Einwirkung auf die Instillationsleitung I, um eine in dieser enthaltene Substanz zum Wundbereich oder zu einer Körperkavität zu befördern.

Bei der in den Figuren 14 bis 18 gezeigten Ausführungsform einer Vorrichtung ist eine Peristaltikkassette 7' als Zwischenteil zwischen einem Fluidsammelbehälter 5` und einem Pumpaggregatgehäuse 1' angeordnet. Die Peristaltikkassette 7', welche somit ein Anschlussteil bildet, weist ein Gehäuse 71' mit einer Schlauchführung für die Instillationsleitung I auf, welche durch das Gehäuse 71' hindurchgeführt ist. Des Weiteren dient die Peristaltikkassette 7` zum Anschliessen einer Sekretleitung S und einer Hilfsleitung H an die Vorrichtung. Vorteilhaft werden im Wesentlichen sämtliche Teile der Peristaltikkassette 7' im Spritzgussverfahren aus einem Kunststoff hergestellt

Die Peristaltikkassette 7' hat insgesamt eine im Wesentlichen quaderförmige, dünne Aussenform. Wenn die Peristaltikkassette 7' bestimmungsgemäss an das Pumpaggregatgehäuse 1' und an den Fluidsammelbehälter 5' angeschlossen ist, wie es in der Figur 14 gezeigt ist, bilden der Fluidsammelbehälter 5`, die Peristaltikkassette 7' und das Pumpaggregatgehäuse 1' gemeinsam eine insgesamt im Wesentlichen quaderförmige Form mit abgerundeten Aussenkanten und -ecken. Die Aussenflächen der Peristaltikkassette 7' sind dann jeweils fluchtend zu den entsprechend angrenzenden Aussenwänden des Pumpaggregatgehäuses 1' angeordnet, also zu einer Vorderwand 10', einer Rückwand, einer ersten Seitenwand 12', einer zweiten Seitenwand, einer oberen Wand 14' und einer unteren Wand. Ausserdem sind die Aussenflächen der Peristaltikkassette 7' jeweils fluchtend zu den entsprechend angrenzenden Aussenwänden des Fluidsammelbehälters 5' angeordnet, also zu einer Vorderwand 50', einer Rückwand, einer ersten Seitenwand 52`, einer zweiten Seitenwand 53`, einer oberen Wand 54` und einer unteren Wand. Die Rückwand, die zweite Seitenwand und die untere Wand des Pumpaggregatgehäuses 1' sowie die Rückwand und die untere Wand des Fluidsammelbehälters 5' sind in den Figuren nicht erkennbar.

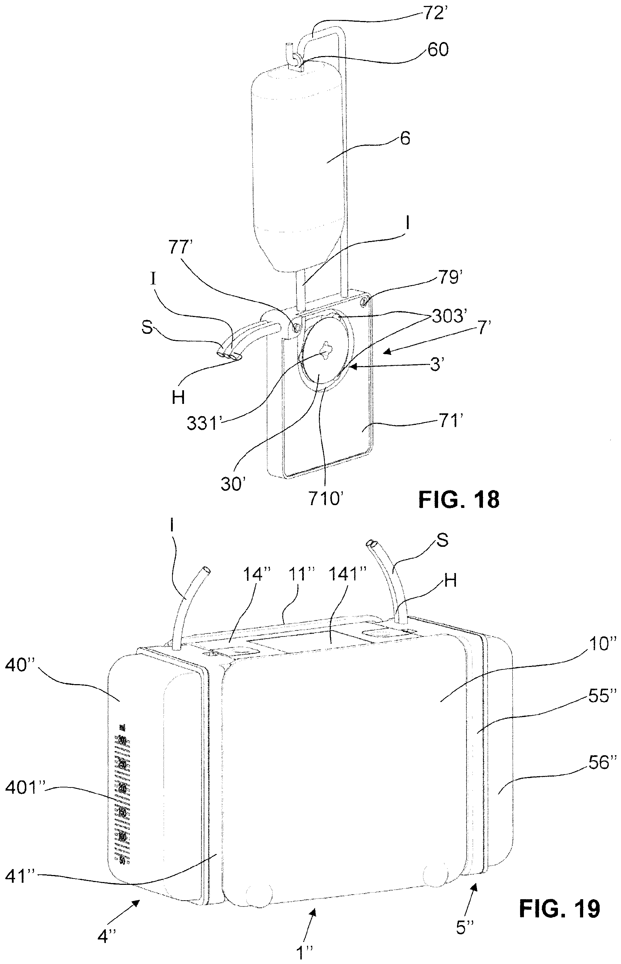

Die Peristaltikkassette 7' verbindet das Pumpaggregatgehäuse 1' und den Fluidsammelbehälter 5' funktionell miteinander, indem es einen gehäuseseitigen Vakuumanschluss 17' des Pumpaggregatgehäuses 1' luftdicht mit einem entsprechend am Fluidsammelbehälter 5' vorgesehenen behälterseitigen Vakuumanschluss 551' verbindet. Die Peristaltikkassette 7' weist hierzu einen dem Fluidsammelbehälter 5' zugewandten Vakuumanschluss 78' und einen dem Pumpaggregatgehäuse 1' zugewandten Vakuumanschluss 79` auf (siehe Figur 15 bzw. 18). Die Vakuumanschlüsse 78' und 79' stehen innerhalb der Peristaltikkassette 7' in fluidkommunizierender Verbindung miteinander.

Die Peristaltikkassette 7' weist ausserdem einen Hilfsanschluss 77' auf (Figur 18), welcher, wenn die Peristaltikkassette 7' bestimmungsgemäss an das Pumpaggregatgehäuse 1' angeschlossen ist, mit einem am Pumpaggregatgehäuse 1' vorgesehenen gehäuseseitigen Hilfsanschluss 181', in fluiddichter Verbindung steht. Der Hilfsanschluss 77' steht innerhalb des Gehäuses 71' in fluidkommunizierender Verbindung mit einem Hilfsleitungsanschluss 74' der Peristaltikkassette 7' (Figur 15), der zum Anschliessen einer Hilfsleitung H dient. Mittels der Hilfsleitung H ist es möglich, bei Bedarf die Sekretleitung S zu spülen und/oder den Druck in der Sekretleitung S zu messen. Die Hilfsleitung H mündet hierzu bevorzugt in der Nähe der Kavität bzw. Wunde in die Sekretleitung S.

Auf ihrer zum Fluidsammelbehälter 5' hin gewandten Seite weist die Peristaltikkassette 7' einen Sekretanschluss 76' auf, welcher zur Ankopplung an einen am Fluidsammelbehälter 5' vorgesehenen behälterseitigen Sekretanschluss 552' ausgebildet ist und innerhalb des Gehäuses 71' in fluidkommunizierender Verbindung mit einem Sekretleitungsanschluss 73' steht. Der Sekretleitungsanschluss 73' dient zum Anschliessen einer Sekretleitung S, um durch diese hindurch via die Sekretanschlüsse 76` und 552' Körperfluide in den Fluidsammelbehälter 5' hinein anzusaugen und in diesem zu sammeln. Im Inneren des Fluidsammelbehälters 5' wird dazu mittels einer im Pumpaggregatgehäuse 1' angeordneten Vakuumpumpe via die Vakuumanschlüsse 17', 79', 78' und 551' ein Vakuum erzeugt.

Auf ihrer dem Pumpaggregatgehäuse 1' zugewandten Seite weist das Gehäuse 71' der Peristaltikkassette 7', wie es in der Figur 18 erkennbar ist, eine im Wesentlichen kreisförmige Vertiefung 710' auf, innerhalb welcher ein Pumpenkopf 30' einer Peristaltikpumpe 3` frei drehbar angeordnet ist. Der Pumpenkopf 30' weist mehrere Anpressrollen 303' auf, welche dazu dienen, auf der in der Vertiefung 710' um den Pumpenkopf 30' herumgelegten Instillationsleitung 1 abzurollen und mittels mechanischer Verformung des Schlauches dabei eine Substanz durch die Instillationsleitung I hindurch dem Wundbereich zuzuführen.

Um die Drehbewegung eines im Pumpaggregatgehäuse 1' untergebrachten Antriebs auf den Pumpenkopf 30' zu übertragen, weist der Pumpenkopf 30' ein zentral angeordnetes Kopplungselement 331' in Form einer Vertiefung auf. Die Vertiefung hat eine nicht kreisrunde Form, welche komplementär zu einem aussenseitig am Pumpaggregatgehäuse 1' angeordneten Kopplungselement 24' ausgebildet ist. Das Kopplungselement 24' ist drehfest an einer Antriebswelle angebracht, welche zur Übertragung einer von dem im Pumpaggregatgehäuse 1' untergebrachten Antrieb bewirkten Drehbewegung auf das Kopplungselement 24' ausgebildet ist.

An der Peristaltikkassette 1' ist ein Aufhängbügel 72' angebracht, um einen mit Instillationsflüssigkeit gefüllten Flüssigkeitsbehälter 6 mittels eines Aufhängers 60 bzgl. der Schwerkraftrichtung oberhalb der Peristaltikkassette 1' aufzuhängen. Die aus dem Flüssigkeitsbehälter 6 herausführende Instillationsleitung I wird durch eine erste Öffnung 75' von oben her in die Peristaltikkassette 7' hinein, innerhalb der Vertiefung 710' um den Pumpenkopf 30' herum und durch eine zweite Öffnung 75' auf der Vorderseite der Peristaltikkassette 7' wieder aus dieser heraus geführt.

Ein Betrieb der in den Figuren 14 bis 18 gezeigten Vorrichtung ist auch ohne die Peristaltikkassette 7' möglich, beispielsweise wenn nur das Absaugen von Körperfluiden nötig ist, nicht jedoch das Zuführen einer Substanz. Der Fluidsammelbehälter 5' wird dazu direkt an das Pumpaggregatgehäuse 1' angeschlossen. Das Kopplungselement 24' ist dann in einer innerhalb der Seitenwand 53` des Fluidsammelbehälters 5` vorgesehenen Vertiefung 57' angeordnet, so dass eine allfällige Drehbewegung des Kopplungselements 24' wirkungslos bleibt. Der gehäuseseitige Vakuumanschluss 17` wird direkt an den behälterseitigen Vakuumanschluss 551' gekoppelt. Zwischen den gehäuseseitigen Hilfsanschluss 181' und den behälterseitigen Sekretanschluss 551' wird ein in den Figuren nicht gezeigter Adapter eingesetzt, welcher einen mit dem Hilfsanschluss 181' verbundenen Hilfsleitungsanschluss sowie einen mit dem Sekretanschluss 551' verbundenen Sekretleitungsanschluss aufweist.

Die Bedienung der in den Figuren 14 bis 18 gezeigten Vorrichtung erfolgt über ein in einem Fenster 141' des Pumpaggregatgehäuses 1' angeordneten Anzeige- und Bedienfeld.

Eine nicht erfindungsgemässe Ausführungsform ist in den Figuren 19 bis 23 gezeigt, bei welcher nicht nur ein Fluidsammelbehälter 5" an einem Pumpaggregatgehäuse 1" fixiert und an dieses angeschlossen werden kann, sondern ein weiteres Anschlussteil in Form eines Instillationsbehälters 4".

Das Pumpaggregatgehäuse 1" ist ähnlich ausgebildet wie dasjenige der Figur 1 und unterscheidet sich von diesem insbesondere dadurch, dass die Vorderwand 10" und die Rückwand 11" mit ihren vertikalen Wandkanten nicht nur zu einer Seite hin, sondern zu beiden Seiten hin, der jeweils dazwischen angeordneten Seitenwand vorstehen. Auf einer Seite des Pumpaggregatgehäuses 1" ist der Fluidsammelbehälter 5" zwischen den Wandkanten der Vorderwand 10" und der Rückwand 11" gehalten und auf der anderen Seite der Instillationsbehälter 4".

Eine obere Wand 14" des Pumpaggregatgehäuses 1" weist ein Fenster 141" für ein Anzeige- und Bedienfeld zur Bedienung und zum Anzeigen des Betriebsstatus der Vorrichtung auf.

Der Fluidsammelbehälter 5" weist eine weitgehend identische Ausgestaltung mit einem Basisteil 55" und einem transparenten Teil 56"auf wie derjenige der in den Figuren 1 bis 7 gezeigten Ausführungsform. Mittels mehrerer Zapfen 412" ist der Fluidsammelbehälter 5" am Pumpaggregatgehäuse 1" befestigbar. Im Unterschied zum Fluidsammelbehälter der Figuren 1 bis 7 ist im vorliegenden Fall jedoch kein Pumpenkopf im Fluidsammelbehälter 5" integriert. Ausserdem werden die Sekretleitung S und die Hilfsleitung H hier durch den Fluidsammelbehälter 5" hindurch nach aussen geführt.

Der Instillationsbehälter 4", welcher vorteilhaft weitgehend vollständig aus einem Kunststoff im Spritzgussverfahren hergestellt ist und zur Bereitstellung einer Instillationsflüssigkeit dient, hat eine ähnliche Ausgestaltung wie der Fluidsammelbehälter 5". Er weist ein transparentes Teil 40", welches mit einer Füllstandskala 401" versehen ist, sowie ein aus einem opaken Material hergestelltes Basisteil 41" auf.

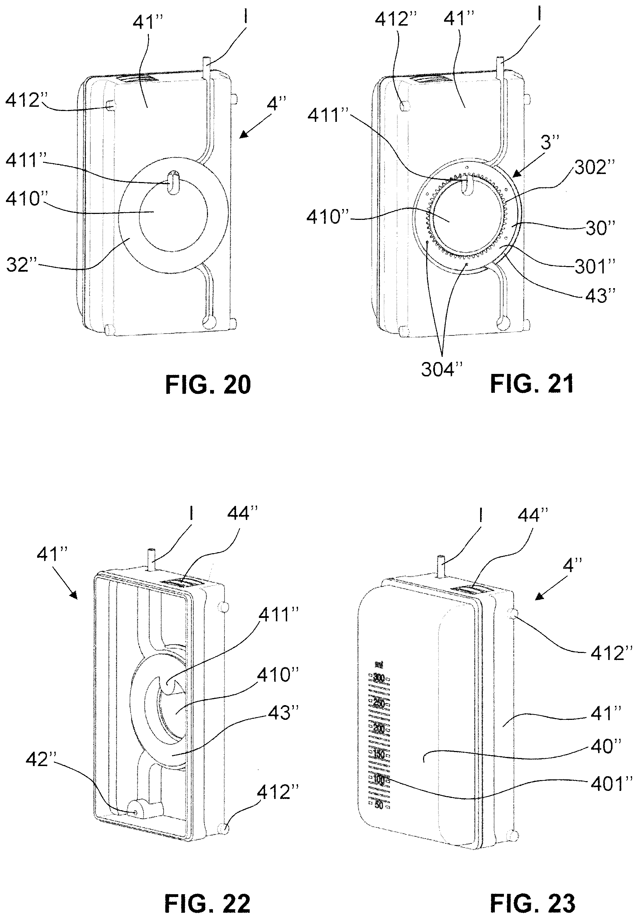

Innerhalb der dem Pumpaggregatgehäuse 1" zugewandten Seitenfläche des Instillationsbehälters 4" ist eine ringförmige Vertiefung 43" ausgebildet, innerhalb welcher ein Hohlrad 301"angeordnet ist (Figur 21). Das Hohlrad 301", an welchem um Rollenachsen 304" drehbare Anpressrollen angebracht sind, bildet einen Pumpenkopf 30" einer Peristaltikpumpe 3".

Das Hohlrad 301" ist frei drehbar an einem konzentrisch innerhalb der ringförmigen Vertiefung 43" vorgesehenen Lagerzapfen 410" angebracht. Um einen kämmenden Eingriff eines in den Figuren nicht sichtbaren Kopplungselements mit einer in radialer Richtung innenseitig am Hohlrad 301" ausgebildeten Verzahnung 302" zu ermöglichen, weist der Lagerzapfen 410" eine in radialer Richtung zur Vertiefung 43" hin offene Aussparung 411" auf. Mittels einer Abdeckung 32" sind die Vertiefung 43" und das Hohlrad 301‴ nach aussen hin abgedeckt.

Eine Instillationsleitung 1, welche via eine Mündung 42" in das Innere des Instillationsbehälters 4" mündet, ist in eine vertieft im Basisteil 41" ausgebildete Schlauchführung eingelegt. Die Schlauchführung wird durch einen sich von der Mündung 42" zur ringfömigen Vertiefung 43" erstreckenden Zuführkanal, der ringförmigen Vertiefung 43" und einen sich von der Vertiefung 43" nach oben hin wegführenden Kanal gebildet. Innerhalb der ringförmigen Vertiefung 43" ist die Installationsleitung 1 derart um den Pumpenkopf 30" herumgelegt, dass bei einer Drehung des Pumpenkopfes 30" die Installationsleitung I mittels der Anpressrollen mechanisch verformt und die Instillationsflüssigkeit dadurch aus dem Instillationsbehälter 4" hinaus und zum Wundbereich hin gepumpt wird.

Am Basisteil 41" ist ausserdem eine Entlüftungsöffnung 44" vorgesehen, um Aussenluft in den Instillationsbehälter 4" einzulassen, wenn im Betrieb der Vorrichtung die Menge an Instillationsflüssigkeit im Instillationsbehälter 4" kleiner wird.

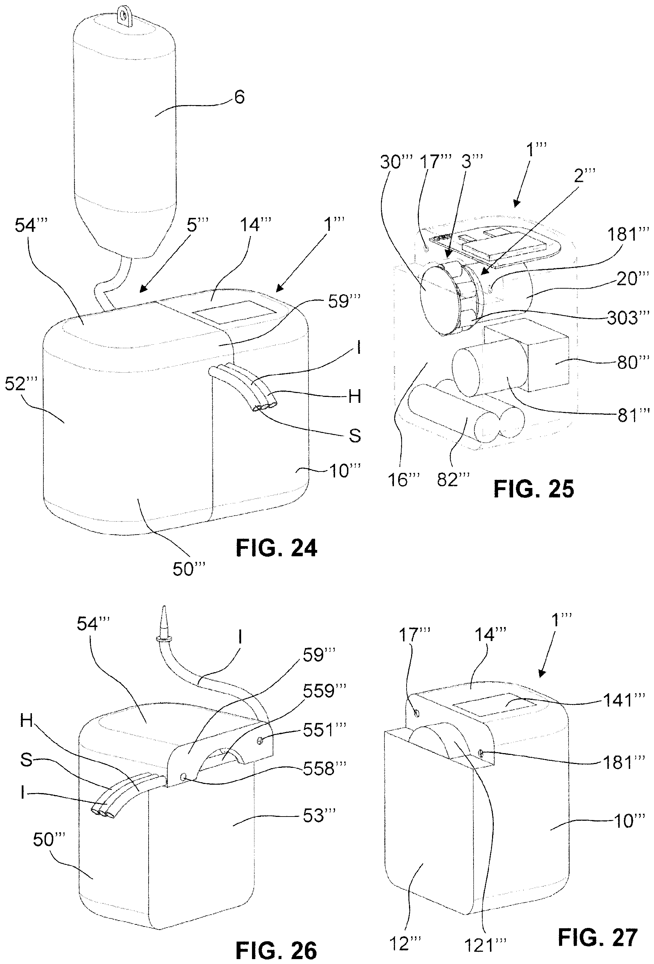

Die Figuren 24 bis 27 zeigen eine weitere Ausführungsform einer erfindungsgemässen Vorrichtung, bei welcher der Pumpenkopf 30‴ einer Peristaltikpumpe 3‴ im Gegensatz zu den in den Figuren 1 bis 23 gezeigten Ausführungsformen nicht in einem Anschlussteil, sondern im Pumpaggregatgehäuse 1‴ integriert ist. Am Pumpaggregatgehäuse 1‴ ist ein Fluidsammelbehälter 5‴ anschliessbar, der eine Schlauchführung 559‴ zur Führung einer Instillationsleitung I aufweist, so dass der in die Schlauchführung 559‴ eingelegte Schlauch in Kombination mit dem Pumpenkopf 30‴ eine Peristaltikpumpe 3‴ bildet.

Wie aus der Figur 25 ersichtlich ist, ist im Innenraum 16‴ des Pumpaggregatgehäuses 1‴ ein Motor 20‴ eines Antriebsstranges 2‴ angeordnet, welcher zum Antreiben des fest an einer Motorwelle angebrachten Pumpenkopfes 30‴ dient. Ausserdem sind im Innenraum 16‴ eine Membranpumpe 80‴ sowie ein Antrieb 81‴ zum Antreiben dieser Membranpumpe 80‴ angeordnet. Die Versorgung der Antriebe 20‴ und 81‴ mit elektrischer Energie erfolgt mittels eines ebenfalls im Innenraum 16‴ angeordneten Akkumulators 82‴.

Die Membranpumpe 80'" dient zum Absaugen von Körperfluiden, indem via einem am Pumpaggregatgehäuse 1‴ vorgesehenen Vakuumanschluss 17‴, der mit einem Vakuumanschluss 551‴ des Fluidsammelbehälters 5‴ in Verbindung steht, ein Vakuum im Fluidsammelbehälter 5'" erzeugt wird. Die Körperfluide werden dadurch durch eine Sekretleitung S hindurch in das Innere des Fluidsammelbehälters 5‴ gesaugt und in diesem gesammelt.

Das Pumpaggregatgehäuse 1‴ weist eine obere Wand 14‴ mit einem Fenster 141'" zur Anordnung eines Anzeige- und Bedienfeldes auf. Eine Seitenwand 12‴ liegt flach am Fluidsammelbehälter 5‴ an, wenn dieser bestimmungsgemäss ans Pumpaggregatgehäuse 1‴ angeschlossen ist.

Das Pumpaggregatgehäuse 1‴ weist zudem einen gehäuseseitigen Hilfsanschluss 181‴ auf, welcher beim Anbringen des Fluidsammelbehälters 5'" an das Pumpaggregatgehäuse 1‴ mit einem am Fluidsammelbehälter 5'" vorgesehenen behälterseitigen Hilfsanschluss 558'" gekoppelt wird, um in fluidkommunizierender Verbindung mit einer Hilfsleitung H zu stehen. Die Hilfsleitung H dient zur Spülung und/oder Messung des Drucks oder der Durchflussmenge der Sekretleitung S.

Der Pumpenkopf 30‴, an welchem auch hier mehrere Anpressrollen 303‴ frei drehbar angebracht sind, ist derart angeordnet, dass zumindest ein Bereich seiner Umfangsfläche im Betrieb eine mechanische Verformung der Instillationsleitung I bewirkt, wenn diese in die Schlauchführung 559'" des Fluidsammelbehälters 5‴ eingelegt ist und der Fluidsammelbehälter 5‴ bestimmungsgemäss am Pumpaggregatgehäuse 1‴ angeschlossen ist. Der Pumpenkopf 30'" kann hierzu mit zumindest einem Teil seiner Umfangsfläche freiliegen, oder, wie es in der vorliegenden Ausführungsform der Fall ist, dort von einer flexiblen Abdeckmembran 121‴ überdeckt sein, um ein Eindringen von Schmutzpartikeln in den Pumpenkopf 30‴ und in den Innenraum 16‴ zu verhindern.

Im Bereich des Pumpenkopfes 30‴ weist das Pumpaggregatgehäuse 1'" eine Stufe auf, deren nach oben hin gewandte Fläche vom Pumpenkopf 30‴ durchragt wird. Die Stufe ermöglicht es, dass der Pumpenkopf 30‴ mit einem Bereich seiner Umfangsfläche auf die in der Schlauchführung 559‴ eingelegte Installationsleitung I einwirken kann. Trotzdem haben das Pumpaggregatgehäuse 1‴ und der bestimmungsgemäss daran angeschlossene Fluidsammelbehälter 5" gemeinsam eine im Wesentlichen quaderförmige Aussengestalt. Aufgrund der Stufe sind die Vorderwand 10‴ und die in den Figuren nicht sichtbare Rückwand des Pumpaggregatgehäuses 1‴ jeweils L-förmig ausgebildet.

Der Fluidsammelbehälter 5‴ weist eine erste Seitenwand 52‴ sowie eine dem Pumpaggregatgehäuse 1‴ zugewandte zweite Seitenwand 53‴ auf, welche von einem oberen vorstehenden Bereich 59‴, der auch eine obere Wand 54‴ bildet, in Richtung zum Pumpaggregatgehäuse 1‴ hin überragt wird. Die Vorderwand 50‴ und die in den Figuren nicht sichtbare Rückwand haben dadurch jeweils die Form eines auf dem Kopf stehenden L.

Auf der nach unten, d.h. zum Pumpenkopf 30‴ hin gewandten Seite des vorstehenden Bereiches 59‴ ist eine kreissegmentartige Ausnehmung vorgesehen, welche das Schlauchbett der Instillationsleitung I in der Peristaltikpumpe 3‴ und somit die Schlauchführung 559‴ bildet. Im Betrieb ist mittels der Peristaltikpumpe 3‴ eine Substanz durch die Instillationsleitung 1 hindurch zum Wundbereich oder zu einer Körperkavität beförderbar.

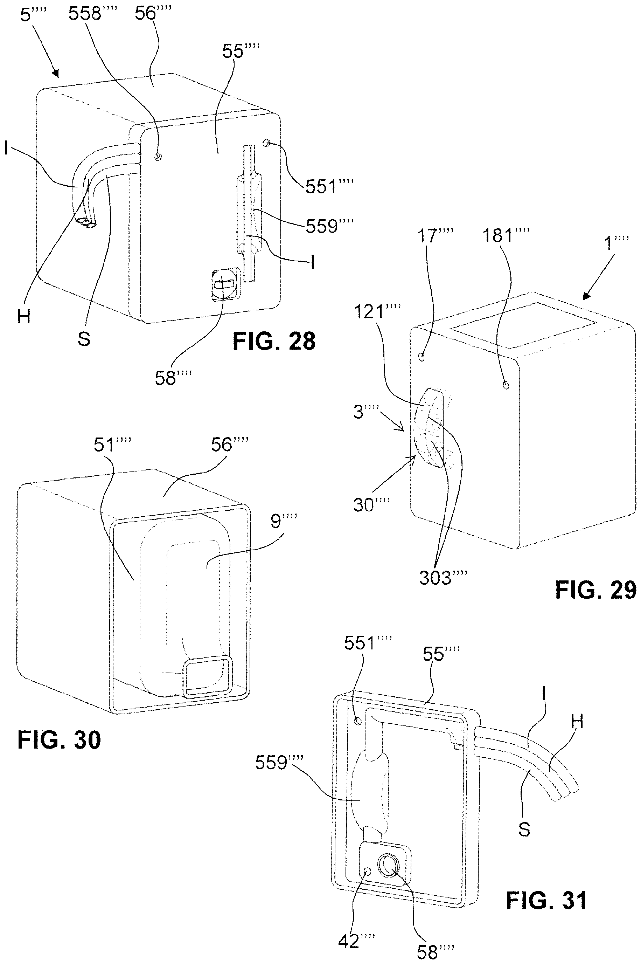

In den Figuren 28 bis 33 ist eine weitere Ausführungsform einer erfindungsgemässen Vorrichtung gezeigt mit einem Pumpaggregatgehäuse 1ʺʺ und einem daran anschliessbaren Anschlussteil in Form eines Fluidsammelbehälters 5"".

Der Fluidsammelbehälter 5"" hat eine quaderförmige Aussengestalt mit einem Basisteil 55ʺʺ und einem vorteilhaft transparenten Teil 56"". Das Basisteil 55"" bildet einen vom transparenten Teil 56ʺʺ abnehmbaren Deckel, welcher die gesamte zum Pumpaggregatgehäuse 1ʺʺ hin gewandte Seitenfläche des Fluidsammelbehälters 5"" bildet. Vorteilhaft ist das Basisteil 55"" als Ganzes und im Wesentlichen vollständig aus einem Kunststoff im Spritzgussverfahren hergestellt.

Die von einer im Pumpaggregatgehäuse 1ʺʺ untergebrachten Vakuumpumpe in das Innere des Fluidsammelbehälters 5"" führende Vakuumleitung erstreckt sich via einen gehäuseseitigen Vakuumanschluss 17"" und einen behälterseitigen Vakuumanschluss 551"" durch das Basisteil 55"" hindurch, wenn der Fluidsammelbehälter 5"" bestimmungsgemäss am Pumpaggregatgehäuse 1ʺʺ angeschlossen ist. Ausserdem mündet eine Sekretleitung S via das Basisteil 55"" in das Innere des Fluidsammelbehälters 5"". Des Weiteren führt eine Hilfsleitung H via einen am Basisteil 55" ausgebildeten behälterseitigen Hilfsanschluss 558ʺʺ und via einen gehäuseseitigen Hilfsanschluss 181"" in das Pumpaggregatgehäuse 1ʺʺ hinein, wenn der Fluidsammelbehälter 5"" am Pumpaggregatgehäuse 1ʺʺ angeschlossen ist. Die Hilfsleitung H dient bei Bedarf zur Spülung und/oder Messung des Drucks der Sekretleitung S.

Das Basisteil 55"" weist auf seiner dem Pumpaggregatgehäuse 1"" zugewandten Seite eine Vertiefung auf, welche vom Schlauch einer Instillationsleitung I durchquert wird. Die Vertiefung bildet das Schlauchbett und somit eine Schlauchführung 559ʺʺ einer Peristaltikpumpe 3"", wenn der Fluidsammelbehälter 5"" bestimmungsgemäss am Pumpaggregatgehäuse 1"" angeschlossen ist.

In dem in der Figur 29 gezeigten Pumpaggregatgehäuse 1ʺʺ ist der Pumpenkopf 30"" der Peristaltikpumpe 3"" dieser Vorrichtung angeordnet, welcher zumindest mit einem Teil seiner Umfangsfläche durch eine entsprechende Seitenwand des Pumpaggregatgehäuses 1ʺʺ hindurch nach aussen zum Fluidsammelbehälter 5ʺʺ hin vorragt. Dieser vorragende Teil des Pumpenkopfes 30"", von dem in der Figur 29 lediglich die Anpressrollen 303ʺʺ gezeichnet sind, kann, wie im vorliegenden Fall, von einer flexiblen Abdeckmembran 121"" abgedeckt sein. Wenn der Fluidsammelbehälter 5"" am Pumpaggregatgehäuse 1ʺʺ angeschlossen ist, ragt der Pumpenkopf 30"" in die die Schlauchführung 559ʺʺ bildende Vertiefung des Basisteils 55"" hinein und presst die Instillationsleitung I dort gegen das Basisteil 55"".

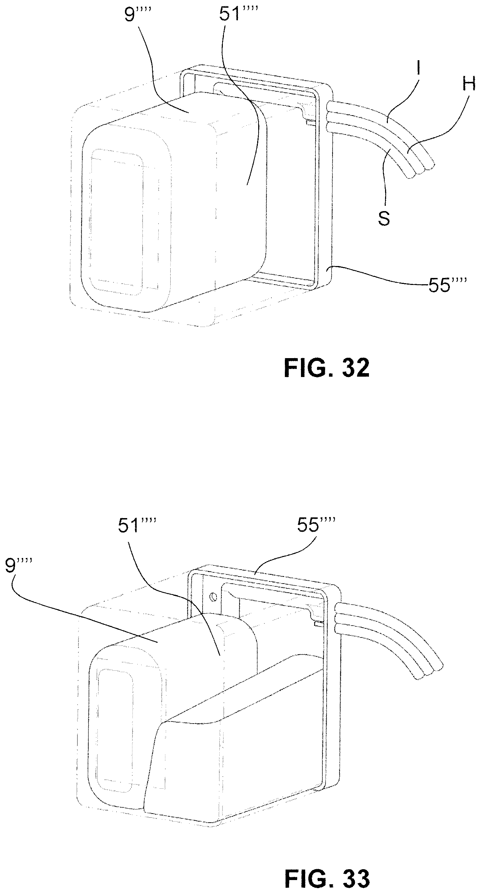

Die in der Schlauchführung 559"" geführte Instillationsleitung I mündet via eine Mündung 42"" in einen im Innenraum 51"" des Fluidsammelbehälters 5"" angeordneten, flexiblen Flüssigkeitsbeutel 9ʺʺ. Im Flüssigkeitsbeutel 9"" ist Instillationsflüssigkeit bereitgestellt, welche mittels der Peristaltikpumpe 3"" durch die Instillationsleitung 1 hindurch dem Körper zuführbar ist. In der Figur 32 ist gezeigt, dass am Anfang einer Behandlung in der Regel nur der Flüssigkeitsbeutel 9"" gefüllt ist. Über die Dauer der Behandlung füllt sich der Fluidsammelbehälter 5"" aber zunehmend mit abgesaugtem Fluid, und das Volumen des Flüssigkeitsbeutels 9"" reduziert sich um das dem Körper zugeführte Fluid..

Mittels eines im Basisteil 55"" angeordneten Verschlusses 58"" kann der Flüssigkeitsbeutel 9ʺʺ entleert oder gefüllt werden, ohne dass das Basisteil 55"" vom transparenten Teil 56ʺʺ abgenommen werden muss.

Bei den in den Pumpaggregatgehäusen 1, 1', 1", 1‴ und 1ʺʺ der in den Figuren 1 bis 33 untergebrachten Antrieben kann es sich insbesondere jeweils um einen bürstenlosen Gleichstrommotor handeln. Ein Akkumulator kann jeweils im Pumpaggregatgehäuse 1, 1', 1", 1‴ bzw. 1"" vorgesehen sein, um den Antrieb mit elektrischer Leistung zu versorgen. Bei allen Ausführungsformen kann der Antriebsstrang zwischen dem Antrieb und dem Pumpenkopf der Peristaltikpumpe ausserdem eines oder mehrere Getriebe aufweisen, um die Drehgeschwindigkeit des Pumpenkopfes gegenüber derjenigen des Antriebs anzupassen. Auch die Anordnung eines Freilaufes im Antriebsstrang ist möglich, um den Pumpenkopf nur dann anzutreiben, wenn der Antrieb eine bestimmte Drehrichtung aufweist. Mittels des zumindest einen Getriebes und/oder Freilaufes können die Peristaltikpumpe und die Vakuumpumpe, wenn beide von einem einzigen gemeinsamen Antrieb antreibbar sind, beliebig mit unterschiedlichen oder gleichen Pumpfrequenzen betrieben werden.

**BEZUGSZEICHENLISTE**

| | | | |
|---|---|---|---|
| 1, 1', 1", 1‴, 1ʺʺ | Pumpaggretatgehäuse | 191 | Rückhaltenase |
| 10`, 10", 10‴ | Vorderwand | 2, 2"' | Antriebsstrang |
| 11, 11" | Rückwand | 20, 20‴ | Motor |
| 12, 12', 12‴ | Seitenwand | 21 | Motorwelle |
| 121‴, 121ʺʺ | Abdeckmembran | 22 | Freilauf/Getriebe |
| 13 | Seitenwand | 23 | Antriebswelle |
| 14, 14', 14", 14‴ | Obere Wand | 24, 24` | Kopplungselement |
| 141, 141', 141", 141‴ | Fenster | | |
| 15 | Untere Wand | 3, 3', 3", 3‴, 3"" | Peristaltikpumpe |
| 16, 16‴ | Innenraum | 30, 30', 30", 30‴, 30"" | Pumpenkopf |
| 17, 17', 17‴, 17ʺʺ | Gehäuseseitiger Vakuumanschluss | 301, 301" | Hohlrad |
| | | 302, 302" | Verzahnung |
| 18 | Adapteraufnahme | 303, 303`, 303‴, 303"" | Anpressrollen |
| 181', 181‴, 181ʺʺ | Gehäuseseitiger Hilfsanschluss | 304, 304" | Rollenachse |
| 19 | Behälteraufnahme | 32, 32" | Abdeckung |
| 190 | Aufnahmehaken | 33 | Innenrad |
| 331, 331' | Kopplungselement | 56, 56", 56"" | Transparentes Teil |
| 34 | Aussenrad | 560 | Füllstandskala |
| | | 57' | Vertiefung |
| 4" | Instillationsbehälter | 58"" | Verschluss |
| 40" | Transparentes Teil | 59‴ | Vorstehender Bereich |
| 401" | Füllstandskala | | |
| 41" | Basisteil | 6 | Flüssigkeitsbehälter |
| 410" | Lagerzapfen | 60 | Aufhänger |
| 411" | Aussparung | | |
| 412" | Zapfen | 7' | Peristaltikkassette |
| 42", 42ʺʺ | Mündung | 71' | Gehäuse |
| 43" | Vertiefung | 710' | Vertiefung |
| 44" | Entlüftungsöffnung | 72' | Aufhängbügel |
| | | 73` | Sekretleitungsanschluss |
| 5, 5`, 5", 5‴, 5"" | Fluidsammelbehälter | 74' | Hilfsleitungsanschluss |
| 50, 50', 50‴ | Vorderwand | 75' | Öffnung |
| 51ʺʺ | Innenraum | 76' | Sekretanschluss |
| 52, 52', 52"` | Seitenwand | 77' | Hilfsanschluss |
| 53, 53`, 53"` | Seitenwand | 78' | Behälterzugewandter Vakuumanschluss |
| 54, 54`, 54'" | Obere Wand | | |
| 55, 55", 55"" | Basisteil | 79' | Pumpaggregatzugewandter Vakuumanschluss |
| 551, 551', 551‴, 551 "" | Behälterseitiger Vakuumanschluss | | |
| | | | |
| | | 80‴ | Membranpumpe |
| 552, 552` | Behälterseitiger Sekretanschluss | 81‴ | Antrieb |
| 553 | Rastkerbe | 82‴ | Akkumulator |
| 554 | Zapfen | | |
| 555 | Vertiefung | 9"" | Flüssigkeitsbeutel |
| 556 | Lagerzapfen | | |
| 557 | Aussparung | S | Sekretleitung |
| 558'", 558"" | Behälterseitiger Hilfsanschluss | H | Hilfsleitung |
| | | I | Instillationsleitung |
| 559‴, 559ʺʺ | Schlauchführung | | |

## Patentansprüche

1. Vorrichtung zum Absaugen von Körperfluiden und zum Zuführen einer fluiden Substanz zu einem menschlichen oder tierischen Körper, aufweisend
eine Antriebseinheit (1, 1', 1‴, 1ʺʺ; 2‴) mit einem Antrieb (20, 20‴),
einen vom Antrieb (20, 20‴) antreibbaren Pumpenkopf (30, 30', 30‴, 30ʺʺ), sowie ein lösbar an die Antriebseinheit (1, 1', 1‴, 1ʺʺ; 2‴) anschliessbares Anschlussteil (5, 5‴, 5ʺʺ, 7') mit einer Schlauchführung (555, 710', 559‴, 559ʺʺ), welche derart zur Aufnahme eines Schlauches (1) ausgebildet ist, dass der Schlauch (1), wenn das Anschlussteil (5, 5‴, 5ʺʺ, 7') bestimmungsgemäss an der Antriebseinheit (1, 1', 1‴, 1ʺʺ; 2‴) angeschlossen ist, in Kombination mit dem Pumpenkopf (30, 30', 30‴, 30ʺʺ) eine Peristaltikpumpe (3, 3', 3‴, 3"") bildet, mit welcher die fluide Substanz durch den Schlauch (1) hindurch zum menschlichen oder tierischen Körper beförderbar ist,
wobei es sich beim Anschlussteil um einen Fluidsammelbehälter (5, 5‴) zum Sammeln der abgesaugten Körperfluide oder um ein Teil (55ʺʺ) eines solchen Fluidsammelbehälters (5ʺʺ) oder um ein Zwischenteil (7') zur Anordnung zwischen der Antriebseinheit (1, 1', 1‴, 1ʺʺ; 2‴) und einem solchen Fluidsammelbehälter handelt,
**dadurch gekennzeichnet, dass**
die Antriebseinheit (1‴, 1ʺʺ; 2‴) ein Gehäuse (1ʺʺ, 1ʺʺ) aufweist, in welchem der Pumpenkopf (30‴, 30"") angeordnet ist, wobei der Pumpenkopf (30‴, 30ʺʺ) zumindest mit einem Teil seiner Umfangsfläche durch eine Seitenwand des Gehäuses (1‴, 1"") hindurch nach aussen zum Anschlussteil (5‴, 5ʺʺ) hin vorragt oder das Gehäuse (1‴) im Bereich des Pumpenkopfes (30‴) eine Stufe mit einer Fläche aufweist, die vom Pumpenkopf (30‴) durchragt wird.

2. Vorrichtung nach einem der vorhergehenden Ansprüche, wobei zumindest einem Teil der Umfangsfläche des Pumpenkopfes (30‴, 30ʺʺ) von einer flexiblen Abdeckmembran (121‴) überdeckt ist.

3. Vorrichtung nach einem der vorhergehenden Ansprüche, wobei das Anschlussteil (5, 5", 5‴, 5ʺʺ, 7') ein identifikationsmerkmal und die Antriebseinheit (1, 1', 1‴, 1ʺʺ; 2‴) eine Identifikationseinheit aufweisen, um zu identifizieren, welche Art eines Anschlussteils (5, 5", 5‴, 5ʺʺ, 7') an der Antriebseinheit (1, 1', 1‴, 1ʺʺ; 2‴) angeschlossen ist, und wobei die Antriebseinheit (1, 1', 1‴, 1ʺʺ; 2‴) dazu ausgebildet ist, in Abhängigkeit der identifizierten Art des Anschlussteils (5, 5", 5‴, 5ʺʺ, 7') einen von mehreren möglichen Betriebsmodi zum Antreiben der Peristaltikpumpe (3, 3', 3‴, 3ʺʺ) auszuwählen.

4. Vorrichtung nach einem der vorhergehenden Ansprüche, wobei es sich beim Anschlussteil (5, 5‴, 5ʺʺ, 7') um ein Wegwerfteil handelt, das für einen einmaligen Gebrauch ausgelegt ist und insbesondere im Wesentlichen vollständig aus Spritzgussteilen hergestellt ist.

5. Vorrichtung nach einem der vorhergehenden Ansprüche, wobei die Antriebseinheit (1, 1', 1‴, 1ʺʺ; 2‴) ausserdem eine Vakuumpumpe, insbesondere eine Membranpumpe (80‴), aufweist, welche zum Absaugen der Körperfluide dient.

6. Vorrichtung nach Anspruch 5, wobei die Antriebseinheit (1, 1', 1‴, 1ʺʺ; 2‴) ein Gehäuse (1, 1', 1‴, 1ʺʺ) aufweist, in welchem sowohl der Antrieb (20‴) als auch die Vakuumpumpe (80‴) angeordnet sind.

7. Vorrichtung nach Anspruch 5 oder 6, wobei derselbe Antrieb (20), welcher zum Antreiben der Peristaltikpumpe (3, 3', 3"") dient, auch zum Antreiben der Vakuumpumpe dient.

8. Vorrichtung nach einem der vorhergehenden Ansprüche, wobei der Pumpenkopf (30, 30', 30‴, 30"") über zumindest einen Freilauf an den Antrieb (20, 20‴) gekoppelt ist.

9. Anschlussteil (5, 5‴, 5ʺʺ, 7') einer Vorrichtung zum Absaugen von Körperfluiden und zum Zuführen einer fluiden Substanz zu einem menschlichen oder tierischen Körper gemäss einem der vorhergehenden Ansprüche, welches lösbar an eine Antriebseinheit (1, 1', 1‴, 1ʺʺ; 2‴) mit einem Antrieb (20, 20‴) anschliessbar ist und aufweist:
eine Schlauchführung (555, 710', 559‴, 559ʺʺ), welche derart zur Aufnahme eines Schlauches (I) ausgebildet ist, dass der Schlauch (I), wenn das Anschlussteil (5, 5‴, 5ʺʺ, 7') bestimmungsgemäss an der Antriebseinheit (1, 1', 1‴, 1ʺʺ; 2‴) angeschlossen ist, in Kombination mit einem vom Antrieb (20, 20‴) antreibbaren Pumpenkopf (30, 30', 30‴, 30"") eine Peristaltikpumpe (3, 3', 3‴, 3"") bildet, mit welcher die fluide Substanz durch den Schlauch (I) hindurch zum menschlichen oder tierischen Körper beförderbar ist, wobei es sich beim Anschlussteil um einen Fluidsammelbehälter (5, 5‴) zum Sammeln der abgesaugten Körperfluide oder um ein Teil (55ʺʺ) eines solchen Fluidsammelbehälters (5ʺʺ) oder um ein Zwischenteil (7') zur Anordnung zwischen der Antriebseinheit (1, 1', 1‴, 1ʺʺ; 2‴) und einem solchen Fluidsammelbehälter handelt, **dadurch gekennzeichnet, dass**
die Antriebseinheit (1‴, 1ʺʺ; 2‴) ein Gehäuse (1ʺʺ, 1ʺʺ) aufweist, in welchem der Pumpenkopf (30‴, 30"") angeordnet ist, und
dass, wenn das Anschlussteil (5, 5‴, 5ʺʺ, 7') lösbar an die Antriebseinheit (1, 1', 1‴, 1ʺʺ; 2‴) angeschlossen ist, der Pumpenkopf (30‴, 30ʺʺ) zumindest mit einem Teil seiner Umfangsfläche durch eine Seitenwand des Gehäuses (1‴, 1ʺʺ) hindurch nach aussen zum Anschlussteil (5‴, 5ʺʺ) hin vorragt oder das Gehäuse (1‴) im Bereich des Pumpenkopfes (30‴) eine Stufe mit einer Fläche aufweist, die vom Pumpenkopf (30‴) durchragt wird.

10. Anschlussteil nach Anspruch 9, wobei es sich beim Anschlussteil um einen Behälter (5ʺʺ) handelt, aufweisend einen Innenraum (51ʺʺ), der in einen ersten Bereich zum Sammeln der abgesaugten Körperfluide sowie in einen zweiten Bereich zur Bereitstellung der fluiden Substanz unterteilt ist, und wobei der erste Bereich des Innenraums (51ʺʺ) mittels einer flexibel ausgebildeten Trennwand vom zweiten Bereich getrennt ist.

11. Anschlussteil (5ʺʺ) nach Anspruch 10, wobei die Trennwand durch einen im Innenraum (51ʺʺ) angeordneten Beutel (9ʺʺ) gebildet wird.

12. Anschlussteil (5ʺʺ) nach einem der Ansprüche 11 oder 12, ausserdem aufweisend:
einen Vakuumanschluss (551"") zum Anschliessen einer Vakuumleitung, um im ersten Bereich des Innenraums (51ʺʺ) ein Vakuum zu erzeugen,
einen Sekretleitungsanschluss zum Anschliessen einer zum menschlichen oder tierischen Körper führenden Sekretleitung (S), um mittels des im ersten Bereich des Innenraums (51ʺʺ) erzeugten Vakuums die Körperfluide durch die Sekretleitung (S) hindurch in den ersten Bereich hinein zu saugen, sowie einen Anschluss zum Anschliessen einer Zuführleitung (I), um die fluide Substanz vom zweiten Bereich des Innenraums (51ʺʺ) aus durch die Zuführleitung hindurch dem menschlichen oder tierischen Körper zuzuführen.

## Claims

1. A device for aspirating body fluids and for supplying a fluid substance to a human or animal body, comprising
a drive unit (1, 1', 1‴, 1ʺʺ; 2‴) having a driver (20, 20‴),
a pump head (30, 30', 30‴, 30ʺʺ) drivable by the driver (20, 20‴), and
a connector (5, 5‴, 5ʺʺ, 7') detachably connectable to the drive unit (1, 1', 1‴, 1ʺʺ; 2‴) and having a hose guide (555, 710', 559‴, 559ʺʺ) configured to receive a hose (1) such that the connector (5, 5‴, 5ʺʺ, 7') when being connected to the drive unit (1, 1', 1‴, 1ʺʺ; 2‴) as intended, in combination with the pump head (30, 30', 30‴, 30ʺʺ) forms a peristaltic pump (3, 3', 3‴, 3ʺʺ) that enables the fluid substance to be delivered to the human or animal body through the hose (1),
wherein the connector is one of
a fluid collection container (5, 5‴) for collecting the aspirated body fluids,
a portion (55ʺʺ) of such a fluid collection container (5ʺʺ), and
an intermediate member (7') for arrangement between the drive unit (1, 1', 1", 1ʺʺ; 2‴) and the fluid collection container,
**characterized in that**
the drive unit (1", 1ʺʺ; 2‴) has a housing (1ʺʺ, 1ʺʺ) in which the pump head (30‴, 30ʺʺ) is arranged, the pump head (30‴, 30ʺʺ) projecting outwardly at least with a part of its circumferential face through a side wall of the housing (1‴, 1ʺʺ) towards the connector (5‴, 5ʺʺ), or in the area of the pump head (30‴) the housing (1‴) has a step with a face penetrated by the pump head (30‴).

2. A device according to any one of the preceding claims, wherein at least a part of the circumferential face of the pump head (30‴, 30ʺʺ) is covered by a flexible covering membrane (121‴).

3. A device according to any one of the preceding claims, wherein the connector (5, 5", 5‴, 5ʺʺ, 7') has an identification feature and the drive unit (1, 1', 1‴, 1ʺʺ; 2‴) has an identification unit configured to identify which type of connector (5, 5", 5‴, 5ʺʺ, 7') is connected to the drive unit (1, 1', 1‴, 1ʺʺ; 2‴), and wherein the drive unit (1, 1', 1‴, 1ʺʺ; 2‴) is configured to select one of a plurality of possible operating modes for driving the peristaltic pump (3, 3', 3‴, 3ʺʺ) depending on the identified type of connector (5, 5", 5‴, 5ʺʺ, 7').

4. A device according to any one of the preceding claims, wherein the connector (5, 5‴, 5ʺʺ, 7') is a disposable component designed for single use and, in particular, is made substantially entirely of injection-molded parts.

5. A device according to any one of the preceding claims, wherein the drive unit (1, 1', 1‴, 1ʺʺ; 2‴) further comprises a vacuum pump, in particular a diaphragm pump (80‴) for aspirating the body fluids.

6. A device according to claim 5, wherein the drive unit (1, 1', 1‴, 1ʺʺ; 2‴) comprises a housing (1, 1', 1‴, 1ʺʺ) accommodating both the driver (20‴) and the vacuum pump (80‴).

7. A device according to claims 5 or 6, wherein the same driver (20) that drives the peristaltic pump (3, 3', 3ʺʺ) also drives the vacuum pump.

8. A device according to any one of the preceding claims, wherein the pump head (30, 30', 30", 30ʺʺ) is coupled to the driver (20, 20‴) via at least one free wheel.

9. A connector (5, 5‴, 5ʺʺ, 7') of a device for aspirating body fluids and for supplying a fluid substance to a human or animal body according to any one of the preceding claims, the connector being detachably connectable to a drive unit (1, 1', 1‴, 1ʺʺ; 2‴) having a driver (20, 20‴), the connector including:
a hose guide (555, 710', 559‴, 559ʺʺ) configured to receive a hose (I) such that the connector (5, 5‴, 5ʺʺ, 7') when being connected to the drive unit (1, 1', 1‴, 1ʺʺ; 2‴) as intended, in combination with the pump head (30, 30', 30‴, 30ʺʺ) forms a peristaltic pump (3, 3', 3‴, 3ʺʺ) that enables the fluid substance to be delivered to the human or animal body through the hose (I), wherein the connector is one of
a fluid collection container (5, 5‴) for collecting the aspirated body fluids,
a portion (55ʺʺ) of such a fluid collection container (5ʺʺ), and
an intermediate member (7') for arrangement between the drive unit (1, 1', 1", 1ʺʺ; 2‴) and the fluid collection container,
**characterized in that**
the drive unit (1", 1ʺʺ; 2‴) has a housing (1ʺʺ, 1ʺʺ) in which the pump head (30‴, 30ʺʺ) is arranged, the pump head (30‴, 30ʺʺ) projecting outwardly at least with a part of its circumferential face through a side wall of the housing (1‴, 1ʺʺ) towards the connector (5‴, 5ʺʺ), or in the area of the pump head (30‴) the housing (1‴) has a step with a face penetrated by the pump head (30‴).

10. A connector according to claim 9, wherein the connector is a container (5ʺʺ) comprising an interior space (51ʺʺ) which is divided into a first region for collecting the aspirated body fluids and a second region for supplying the fluid substance, and wherein the first region of the interior space (51ʺʺ) is separated from the second region by means of a flexibly formed separating wall.

11. A connector (5ʺʺ) according to claim 10, wherein the separating wall is formed by a pocket (9ʺʺ) provided in the interior space (51ʺʺ).

12. A connector (5ʺʺ) according to any one of claims 11 or 12, further comprising:
a vacuum joint (551ʺʺ) for connecting a vacuum line to create a vacuum in the first region of the interior space (51ʺʺ),
a secretion line connector for connecting a secretion line (S) leading to the human or animal body for aspirating the body fluids through the secretion line (S) into the first region by means of the vacuum generated in the first region of the interior space (51ʺʺ), and
a supply line connector (I) for supplying the fluid substance from the second region of the interior space (51ʺʺ) through the supply line to the human or animal body.

## Revendications

1. Dispositif pour aspirer des fluides corporels et pour administrer une substance fluide à un corps humain ou animal, comprenant
une unité d'entraînement (1, 1', 1''', 2ʺʺ ; 2‴) avec un entraînement (20, 20‴),
une tête de pompe (30, 30', 30''', 30'''') qui peut être entraînée par l'entraînement (20, 20‴), et
une partie de connexion (5, 5‴, 5ʺʺ, 7') qui peut être connectée de manière amovible à l'unité d'entraînement (1, 1', 1‴, 1ʺʺ ; 2‴) et qui comporte un guide de tuyau (555, 710', 559‴, 559'''') conçu pour maintenir un tuyau (I) de telle sorte que, quand la partie de connexion (5, 5‴, 5ʺʺ, 7') est connectée à l'unité d'entraînement (1, 1', 1‴, 1ʺʺ ; 2‴) comme prévu, le tuyau (I) forme en combinaison avec la tête de pompe (30, 30', 30''', 30'''') une pompe péristaltique (3, 3', 3‴, 3'''') avec laquelle la substance fluide peut être administrée à travers le tuyau (I) au corps humain ou animal,
dans lequel la partie de connexion est un récipient de collecte de fluide (5, 5‴) pour collecter les fluides corporels aspirés, ou une partie (55'''') d'un tel récipient de collecte de fluide (5ʺʺ), ou une partie intermédiaire (7') destinée à être agencée entre l'unité d'entraînement (1, 1', 1‴, 1ʺʺ ; 2‴) et un tel récipient de collecte de fluide,
**caractérisé en ce que** l'unité d'entraînement (1''', 1ʺʺ ; 2''') comporte un boîtier (1‴, 1ʺʺ) dans lequel la tête de pompe (30''', 30'''') est disposée, dans lequel au moins une partie de la surface périphérique de la tête de pompe (30‴, 30'''') traverse une paroi latérale du boîtier (1‴, 1ʺʺ) vers l'extérieur jusqu'à la partie de connexion (5‴, 5ʺʺ), ou dans lequel le boîtier (1‴) comporte dans la région de la tête de pompe (30‴) une protubérance avec une surface à travers laquelle dépasse la tête de pompe (30‴).

2. Dispositif selon l'une des revendications précédentes, dans lequel au moins une partie de la surface périphérique de la tête de pompe (30‴, 30'''') est recouverte par une membrane de revêtement flexible (121‴).

3. Dispositif selon l'une des revendications précédentes, dans lequel la partie de connexion (5, 5", 5‴, 5ʺʺ, 7') comporte un élément d'identification et l'unité d'entraînement (1, 1', 1‴, 1ʺʺ ; 2''') comporte une unité d'identification afin d'identifier le type de la partie de connexion (5, 5", 5‴, 5ʺʺ, 7') connectée à l'unité d'entraînement (1, 1', 1‴, 1ʺʺ ; 2'''), et dans lequel l'unité d'entraînement (1, 1', 1‴, 1ʺʺ ; 2''') est conçue pour sélectionner un mode parmi plusieurs modes de fonctionnement possibles pour l'entraînement de la pompe péristaltique (3, 3', 3‴, 3ʺʺ) en fonction du type de partie de connexion (5, 5", 5‴, 5ʺʺ, 7') identifié.

4. Dispositif selon l'une des revendications précédentes, dans lequel la partie de connexion (5, 5‴, 5ʺʺ, 7') est une pièce à usage unique conçue pour une seule utilisation, et en particulier constituée essentiellement dans son entièreté à partir de pièces moulées.

5. Dispositif selon l'une des revendications précédentes, dans lequel l'unité d'entraînement (1, 1', 1''', 1ʺʺ ; 2‴) comporte en outre une pompe à vide, en particulier une pompe à membrane (80‴), qui sert à aspirer les fluides corporels.

6. Dispositif selon la revendication 5, dans lequel l'unité d'entraînement (1, 1', 1‴, 1ʺʺ ; 2''') comporte un boîtier (1, 1', 1''', 1'''') dans lequel sont agencés à la fois l'entraînement (20‴) et la pompe à vide (80‴).

7. Dispositif selon la revendication 5 ou 6, dans lequel l'entraînement (20) utilisé pour entraîner la pompe péristaltique (3, 3', 3ʺʺ) est également utilisé pour entraîner la pompe à vide.

8. Dispositif selon l'une des revendications précédentes, dans lequel la tête de pompe (30, 30', 30‴, 30ʺʺ) est couplée à l'entraînement (20, 20‴) par l'intermédiaire d'au moins une roue libre.

9. Partie de connexion (5, 5‴, 5ʺʺ, 7') d'un dispositif d'aspiration de fluides corporels et d'administration d'une substance fluide à un corps humain ou animal selon l'une des revendications précédentes, qui peut être connectée de manière amovible à une unité d'entraînement (1, 1', 1‴, 1ʺʺ ; 2''') via à un entraînement (20, 20''') et comporte :
un guide de tuyau (555, 710', 559‴, 559ʺʺ) qui est conçu pour recevoir un tuyau (I) de telle sorte que, lorsque la partie de connexion (5, 5''', 5ʺʺ, 7') est connectée comme prévu à l'unité d'entraînement (1, 1', 1‴, 1ʺʺ ; 2‴), le tuyau (I) constitue conjointement à une tête de pompe (30, 30', 30‴, 30ʺʺ) entraînable par un entraînement (20, 20‴) une pompe péristaltique (3, 3', 3‴, 3ʺʺ), avec laquelle la substance fluide peut être administrée à travers le tuyau (I) au corps humain ou animal, dans laquelle la partie de connexion est un récipient de collecte de fluide (5, 5‴) pour collecter les fluides corporels aspirés, ou une partie (55ʺʺ) d'un tel récipient de collecte de fluide (5ʺʺ), ou une partie intermédiaire (7') destinée à être agencée entre l'unité d'entraînement (1, 1', 1‴, 1ʺʺ ; 2‴) et un tel récipient de collecte de fluide,
**caractérisée**
**en ce que** l'unité d'entraînement (1''', 1ʺʺ ; 2‴) comporte un boîtier (1''', 1'''') dans lequel la tête de pompe (30‴, 30'''') est disposée, et
**en ce que** quand la partie de connexion (5, 5‴, 5ʺʺ, 7') est connectée de manière amovible à l'unité d'entraînement (1, 1', 1‴, 1ʺʺ ; 2'''), au moins une partie de la surface périphérique de la tête de pompe (30''', 30ʺʺ) traverse une paroi latérale du boîtier (1‴, 1ʺʺ) vers l'extérieur jusqu'à la partie de connexion (5‴, 5ʺʺ), ou le boîtier (1''') comporte dans la région de la tête de pompe (30''') une protubérance avec une surface à travers laquelle dépasse la pompe la tête (30‴).

10. Partie de connexion selon la revendication 9, dans laquelle la partie de connexion est un récipient (5ʺʺ) comportant un espace interne (51'''') qui se divise en une première région pour collecter les fluides corporels aspirés et une deuxième région pour fournir la substance fluide, et dans laquelle la première région de l'espace interne (51'''') est séparée de la deuxième région au moyen d'une cloison de séparation flexible.

11. Partie de connexion (5ʺʺ) selon la revendication 10, dans laquelle la cloison de séparation est formée par une poche (9ʺʺ) disposée dans l'espace interne 51ʺʺ).

12. Partie de connexion (5ʺʺ) selon l'une des revendications 11 ou 12, comprenant en outre :
une connexion sous vide (551ʺʺ) pour connecter une conduite de vide afin de générer un vide dans la première région de l'espace interne (51ʺʺ),
une connexion de conduite de sécrétion pour connecter une conduite de sécrétion (S) menant au corps humain ou animal afin d'aspirer les fluides corporels à travers la conduite de sécrétion (S) au moyen du vide généré dans la première région de l'espace interne (51''''), et une connexion pour connecter une conduite d'administration (I) afin d'administrer la substance fluide provenant de la deuxième région de l'espace interne (51'''') à travers la conduite d'administration au corps humain ou animal.
